# EUROPEAN PATENT SPECIFICATION

(11) **EP 1 688 393 B1**
(45) Date of publication and mention of the grant of the patent: **11.01.2012**
(21) Application number: 04818561.5
(22) Date of filing: 17.11.2004
(51) Int. Cl.: C01B 39/46, B01J 29/70, C07D 201/04, C07D 223/10

(54) **HIGH SILICA CDS-1 ZEOLITE**
SILICIUMREICHER ZEOLITH CDS-1
ZEOLITE CDS-1 RICHE EN SILICE

(30) Priority: 17.11.2003 JP 2003386809; 17.11.2003 JP 2003387299; 26.12.2003 JP 2003435651
(43) Date of publication of application: 09.08.2006
(73) Proprietor: National Institute of Advanced Industrial Science and Technology, Tokyo 100-8921 (JP)
(72) Inventor: IKEDA, Takuji, N. Inst. of Adv. Ind. Sci. & Tech., Sendai-shi, Migayi 9838551 (JP); KOMURA, Kenichi, N. Inst. of Adv. Ind. Sci. & Tech, Sendai-shi, Migayi 983-8551 (JP); MIZUKAMI, Fujio, N. Inst. of Adv. Ind. Sci. & Tech, Sendai-shi, Migayi 9838551 (JP); NIWA, Syuichi, N. Inst. of Adv. Ind. Sci. & Tech., Miyagino-ku, Sendai-shi, Migayi 983-8551 (JP); YOKOYAMA, Toshirou, N. Inst. of Adv. Sci. & Tech., Sendai-shi, Miyagi 9838551 (JP); HANAOKA, Takaaki, N. Inst. of Adv. Sci. & Tech., Sendai-shi, Miyagi 9838551 (JP); SATO, Koichi, N. Inst. of Adv. Sci. & Technology, Sendai-shi, Miyagi 9838551 (JP); KIYOZUMI, Yoshimichi, N. Inst of Adv. Sci. & Tech., Sendai-shi, Miyagi 9838551 (JP); HASEGAWA, Yasuhisa, N. Inst. of Adv. Sci. & Tech., Sendai-shi, Miyagi 9838551 (JP); MANICKAM, Sasidharan, N. Inst. of Adv. Sci. & Tech, Sendai-shi, Miyagi 9838551 (JP)
(74) Representative: Ebner von Eschenbach, Jennifer
(86) International application number: PCT/JP2004/017106
(87) International publication number: WO 2005/047182

(56) References cited:
- JP-A- 7 291 620
- JP-A- 2003 073 116
- JP-A- 2004 175 661
- JP-A- 2004 339 044
- ADALGISA TAVOLARO , ENRICO DRIOLI: "zeolite membranes" ADVANCED MATERIALS, vol. 11, no. 12, 18 August 1999 (1999-08-18), pages 975-996, XP002520325 Wiley-VCH VErlag GmbH & Co, Weinheim
- A. CORMA1, V. FORNES1, S. B. PERGHER1, TH. L. M. MAESEN2 & J. G. BUGLASS3: "Delaminated zeolite precursors as selective acidic catalysts" LETTERS TO NATURE, vol. 396, 26 November 1998 (1998-11-26), pages 353-356, XP002520326 doi:10.1038/24592
- A. CORMA, V. FORNÉS, J. MARTÍNEZ-TRIGUERO AND S. B. PERGHER: "Delaminated Zeolites: Combining the Benefits of Zeolites and Mesoporous Materials for Catalytic Uses" JOURNAL OF CATALYSIS, vol. 186, no. 1, 15 August 1999 (1999-08-15), pages 57-63, XP002520327 doi:10.1006/jcat.1999.2503
- T. IKEDA, Y. AKIYAMA, F. IZUMI, Y. KIYOZUMI, F. MIZUKAMI,AND T. KODAIRA: "Crystal Structure of a Helix Layered Silicate Containing Tetramethylammonium Ions in Sodalite-Like Cages" CHEMISTRY OF MATERIALS, vol. 13, no. 4, 13 March 2001 (2001-03-13), pages 1286-1295, XP002520328 DOI: 10.1021/cm000736v
- TAKUJI IKEDA, DR., YOSHIKATSU AKIYAMA, DR., YASUNORI OUMI, DR., AKIKO KAWAI, DR., FUJIO MIZUKAMI, DR.: "The Topotactic Conversion of a Novel Layered Silicate into a New Framework Zeolite" ANGEWANDTE CHEMIE INTERNATIONAL EDITION, vol. 43, no. 37, 16 September 2004 (2004-09-16), pages 4892-4896, XP002520329 10.1002/anie.200460168

## Description

### TECHNICAL FIELD

The present invention relates to a crystalline layered compound which can be used in solids for metal carrier, precursor materials for synthesizing novel zeolites, separation and adsorption agents, shape-selective solid catalysts, ion exchange agents, chromatography fillers and the like, to a novel high silica content zeolite which can be used in separation and adsorption agents, shape-selective solid catalysts, ion exchange agents, chromatography fillers, chemical reaction sites and the like, and to methods of manufacturing these and the like.

### BACKGROUND ART

Zeolites have micropores (3 to 10 Å) regularly arranged on a atomic level, and the aluminosilicate, which has a skeleton consisting of the elements Si, Al and O has chemical and physical adsorption effects due to shape-selective or the skeletal structure, has function as molecular sieves, separation and adsorption agents, ion exchange agents and petroleum-related catalysts. About 150 different structures are known as natural and synthetic zeolites, and selecting the composition of the structural elements allows them to be applied to a wide range of petrochemical and other industrial fields as porous materials with chemical properties, structural stability and heat resistance suited to such purposes.

Each zeolite is distinguished by a crystal structure with regular pores, yielding a definitive X-ray diffraction pattern. The crystal structure defines the shape and size of the zeolite pores and holes. The adsorption properties and catalytic functions of each molecular sieve are partially determined by the shape and size of its pores and holes. Consequently, when considering a particular application, the usefulness of a particular zeolite is at least partially dependent on its crystal structure. The zeolites in actual use can be classified structurally into only 10 types. This is because many do not have sufficient mechanical strength or heat resistance for actual use.

High-silica composition zeolites have sufficient mechanical strength and are superior to low-silica compositions in having high heat resistance and high hydrophobicity. These properties are important when zeolites are used as catalysts in organic reactions. In the early stages of zeolite synthesis research it was only possible to obtain products with low silica/alumina ratios, but the synthesis of a zeolites with a high silica/alumina ratio composition was made possible by the addition of an organic structure directing agent to a starting gel comprising the silica source (R. M. Barrer, 1982, Hydrothermal Chemistry of Zeolites, New York: Academic Press, Inc., pp. 157 to 170). For example, silicalite which is an MFI-type zeolite is highly hydrophobic and is used as a separation and adsorption agent.

The catalytic functions and adsorption properties of a zeolite are dependent on the size and shape of the pores and holes or in other words on its crystal structure. At present, only a limited number of zeolites can be used industrially, and zeolites with novel crystal structures need to be synthesized in order to expand the range of applications. In particular, if high-silica zeolites with novel structures and excellent heat resistance could be synthesized they would be extremely useful as high-function catalysts for vapor-phase Beckmann rearrangement for example, which has become a focus of attention in recent years (Japanese Patent Applications Laid-open Nos. 2000-256308, 2000-256309).

These zeolites are normally prepared under self-pressure by hydrothermal synthesis, in which a large quantity of water, an aluminum source, a silica source, an alkali metal and an amine or other organic structure directing agent (template agent for forming pores in the resulting zeolite) are mixed together to form the desired chemical composition, which is then sealed inside an autoclave or other pressure container. In recent years, the synthesis of high-silica zeolites with larger pore sizes has become an issue in the field of catalysts and materials.

One approach to this is to increase the spatial size of the organic structure directing agent in order to increase the pore size of the resulting zeolite. At present, organic structure directing agents are believed to play a vital roll in the zeolite crystallization process. Organic amines and quaternary ammonium cations were first used as the directing agents in the early 1960's (R. M. Barrer and P. J. Denny, J. Chem. Soc. 1961, 971 to 982). Not only have many novel zeolites been discovered through this approach, but the range of chemical compositions of the crystalline products has also increased.

With the exception of some quaternary ammonium cations and the like, however, molecular design guideline for forming zeolite frameworks have not been established using most organic structure directing agents, and synthesis requires time and a high level of technology. For industrial purposes, a process of baking these organic structure directing agents is required to remove them from the zeolite, raising environmental and cost issues. Moreover, the mechanism of crystallization in hydrothermal synthesis is still not understood.

Meanwhile, zeolite membranes comprising such a zeolite (powder) formed as a membrane on a ceramic or metal porous base have come to be used for separating alcohol and water by permeance-vaporization method, and as gas separation membranes by using the molecular sieve function and affinities (hydrophobicity or hydrophilicity) of the zeolites. Under these circumstances, various zeolite membranes using porous supports have been proposed along with manufacturing methods therefor.

Recent improvements in zeolite membrane synthesis technology have led to practical applications in the area of alcohol separation methods other than distillation, such as for example alcohol concentration by means of selective water permeation from an alcohol-water solution using the hydrophilicity of an A-type zeolite having an oxygen 8-member ring structure (Japanese Patent Application Laid-open No. H07-185275). However, this A-type zeolite is less acid resistant than other high-silica zeolites (its structure is destroyed by contact with acid), making it difficult to use for separating water from an acidic mixture.

High-silica composition zeolites are superior to A-type zeolites and other low-silica composition membranes in terms of both heat resistance and hydrophobicity. For this reason, various studies have been aimed at developing methods of manufacturing high-silica zeolite membranes.

Because zeolites have low plasticity, in most cases they are made into membranes by hydrothermal synthesis, in which a large quantity of water, an aluminum source, a silica source, an alkaline metal and an amine or other organic structure directing agent are mixed together to form a zeolite composition for a particular purpose, and then sealed inside an autoclave or other pressure container and heated in the presence of an alumina, mullite or other porous base or tube to synthesis a zeolite membrane on that base. Hydrothermal synthesis can also be performed after zeolite seed crystals have already been applied to synthesize defect-free zeolite membranes (for example, Japanese Patent Application Laid-open No. 2003-159518: Method for Manufacturing DDR Type Zeolite Membrane). Zeolite membranes synthesized by these techniques are used in separation and concentration from mixtures gas and liquid mixtures (for example, Japanese Patent Application Laid-open No. 2003-144871, Mordenite Type Zeolite Film Composite and Method for Manufacturing the Same and Concentrating Method Using the Composite). However, these zeolite membranes are all prepared using existing zeolites, and there is a strong demand in the field for the development of still better zeolite membranes.

Meanwhile, ε-caprolactam is a principal raw material in 6-nylon manufacture, and a staple substance in organic chemical engineering. The primary industrial method of manufacturing ε-caprolactam is the so-called Beckmann rearrangement reaction, in which ε-caprolactam is obtained by rearrangement of cyclohexanone oxime in a liquid-phase reaction using a sulfuric acid catalyst. Methods have also been studied of using solid acids as the catalyst in place of sulfuric acid. These reactions are performed in a gaseous phase, and boric acid based catalysts (Japanese Patent Applications Laid-open Nos. S53-37686 and S46-12125), silica-alumina based catalysts (GB Patent 881927, Specifications), solid phosphoric acid catalysts (GB Patent 881926), composite metal oxide catalysts (Nihon Kagaku Kaishi No. 1, 77(1977) and zeolite based catalyst (Journal of Catalysis 6, 247(1966) and Japanese Patent Application Laid-open No. S57-139062) have been proposed. More recently, low-solid-acid silica zeolite catalysts having few of the properties of solid acids have also been studied (Japanese Patent Applications Laid-open Nos. S62-126167, S63-54358 and S62-281856) based.

Because a large quantity of fuming sulfuric acid is used in the aforementioned method using sulfuric acid, constant corrosion of the equipment is a problem, and a greater problem is disposing of the large amount of ammonium sulfate produced as a bi-product. Methods using solid acids have been proposed for solving these problems as discussed above, but in all cases there are problems with the selection rate of the target ε-caprolactam, the catalyst life and the like. Recently a Beckmann rearrangement reaction using low-solid-acid silica zeolite (MFI-type) has been discovered, which is reported to be highly selective. The low-solid-acid silica zeolite in this case has a high silica content (silicalite or ZSM-5) based.

The problem, however, is that in general large quantities of extremely expensive amines such as TPAOH (tetrapropylammonium hydroxylate) must be used as structure directing agents in the preparation of such membranes. Moreover, synthesis is under hydrothermal conditions, which are not only complex but necessitate an operation of baking of the amine when the zeolite is used, resulting in a large expenditure of thermal energy. Complex procedures must also be repeated to remove the aluminum in synthesizing this low-aluminum type of silica zeolite, and the catalysts are very expensive. Under these circumstances, there is strong demand for the development of zeolite catalysts which are highly functional in Beckmann rearrangement and which do not require complex manufacturing steps or expensive amines.

### DISCLOSURE OF THE INVENTION

Since conventional hydrothermal synthesis methods involve one-stage synthesis, various synthesis conditions need to be precisely tested and adjusted in order to synthesize zeolites with novel crystal structures, and it is particularly difficult to create skeletal frameworks as desired.

This problem is attributed to the fact that no clear design processes have been established for substance design in conventional synthesis methods. One idea for manufacturing new zeolite compounds with various functions is to design and organize novel compounds using the basic skeletal parts of layered compounds that are highly organized on an atomic level, as though using building blocks. However, there have heretofore been no theoretical methods for synthesizing only the desired parts and efficiently linking them together. The inventors in this case devised and perfected the present invention in the course of research into solid-phase transition reactions from amorphous silica-alumina and layered compounds into zeolite.

It is an object of the first aspect of the present invention to provide, in the synthesis of a high-silica zeolite membrane, a method for easily synthesizing a CDS-1 zeolite membrane that is not an existing zeolite but an entirely new zeolite. It is also an object of the present invention to provide a method for synthesizing a dense CDS-1 membrane. It is another object of the present invention to provide a novel zeolite membrane having the properties of high hydrophobicity and excellent heat resistance. It is also an object of the present invention not only to allow the easy and quick manufacture of a zeolite membrane which is adaptable to industrial liquid and gas separation processes and the like, but to provide a zeolite membrane that is suitable as a membrane filter with both separation and catalytic functions in the petrochemical industry for example.

Moreover, it is an object of the second aspect of the resent invention to provide a method for producing ε-caprolactam by Beckmann rearrangement reaction from cyclohexane oxime using CDS-1 as the catalyst. It is also an object of the present invention to provide a novel method for industrial production of ε-caprolactam, wherein ε-caprolactam is synthesized highly efficiently using CDS-1, a novel high-silica zeolite catalyst which is highly functional in Beckmann rearrangement.

An aspect outside of the present invention is explained below.

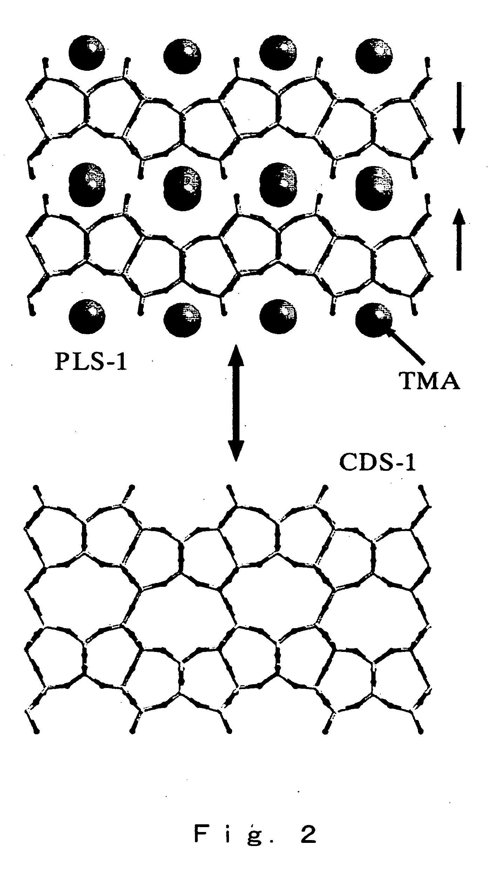

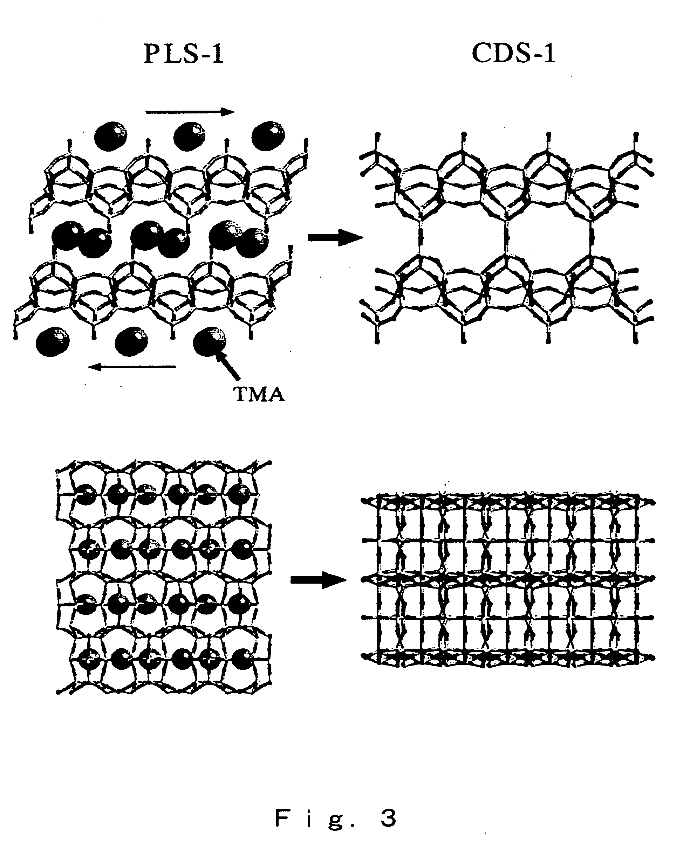

Focusing on the similarity between the basic structures of crystalline layered compounds and many zeolite structures, the inventors perfected the present invention upon discovering that a zeolite having a crystal structure geometrically similar to that of a layered compound such as that shown in Figures 2 and 3 and exhibiting a novel powder x-ray diffraction pattern could be synthesized by phase transition techniques using dehydration polycondensation. This layered compound is called PLS-1 (Pentagonal-cylinder Layered Silicate), while the novel zeolite obtained from PLS-1 is called CDS-1 (Cylindrically Double Saw-Edged type 1).

That is:
(1) A crystalline layered compound characterized in that the chemical composition of which is represented by [(Si₁₈₋ₓ·O₃₈) ·M_{y}· (TMA)_{z} · (H₂O) _{w}] (wherein TMA is a tetraalkylammonium cation, M is a cation of an alkali metal such as Na, K or Li, x satisfies 0 < x ≤ 1.2, y satisfies 0.5 ≤ y ≤ 1.5, z satisfies 6 ≤ z ≤ 8 and w satisfies 0.02 ≤ w ≤ 1.5), having as the basic structure thereof a single-layer skeleton comprising one-dimensional micropores nanometers in size formed by a network of covalent bonds between Si and O atoms, the lattice spacing d in the powder x-ray diffraction pattern being at least as described in Table 7 below (wherein d is the lattice spacing, w = weak relative strength, m = moderate relative strength, s = strong relative strength and vs = extremely strong relative strength).

**Table 7**

| d(Å) | Relative strength |
|---|---|
| 10.47±0.2 | vs |
| 8.38±0.15 | w |
| 7.34±0.15 | m |
| 7.00±0.1 | m |
| 6.51±0.1 | m |
| 6.45±0.1 | s |
| 5.86±0.05 | m |
| 5.82±0.04 | m |
| 5.66±0.04 | w |
| 5.23±0.04 | m |
| 5.07±0.04 | w |
| 4.90±0.04 | s |
| 4.75±0.04 | m |
| 4.57±0.04 | w |
| 4.40±0.04 | m |
| 4.35±0.04 | s |
| 4.26±0.04 | s |
| 9.19±0.04 | vs |
| 4.00±0.04 | m |
| 3.94±0.035 | s |
| 3.85±0.035 | s |
| 3.83±0.035 | vs |
| 3.78±0.035 | w |
| 3.67±0.035 | m |
| 3.63±0.035 | s |
| 3.60±0.035 | w |
| 3.55±0.035 | m |
| 3.51±0.035 | m |
| 3.50±0.035 | vs |
| 3.48±0.035 | vs |
| 3.38±0.035 | m |
| 3.34±0.035 | w |
| 3.32±0.035 | s |

(2) The crystalline layered compound according to (1) above, wherein in the aforementioned layered compound the local coordination of the O atoms surrounding the Si atoms in the Si-O network is tricoordinate and tetracoordinate.
(3) The crystalline layered compound according to (1) above, wherein in the aforementioned layered compound alkali metal cations and an organic structure directing agent are included in the gaps between layers of the crystal structure.
(4) The crystalline layered compound according to (1) above, wherein in the aforementioned layered compound the effective gap between layers is 3 Å or more.
(5) The crystalline layered compound according to (1) above, wherein the aforementioned layered compound has pores formed of skeletal sites which are silicon 5-member rings or larger.
(6) A method for manufacturing a crystalline layered compound characterized by comprising heating a crystalline layered compound in the presence of an organic structure directing agent, to synthesize a crystalline layered compound with the chemical composition represented by [ (Si₁₈₋ₓ·O₃₈) ·M_{y}·(TMA)_{z} · (H₂O)_{w}] (wherein TMA is a tetraalkylammonium cation, M is a cation of an alkali metal such as Na, K or Li, x satisfies 0 ≤ x ≤ 1.2, y satisfies 0.5 ≤ y ≤ 1.5, z satisfies 6 ≤ z ≤ 8 and w satisfies 0.02 ≤ w ≤ 1.5).
(7) The method for manufacturing a crystalline layered compound according to (6) above, wherein a crystalline layered compound defined in any of (1) to (5) above is synthesized.
(8) The method for manufacturing a crystalline layered compound according to (6) or (7) above, wherein the organic structure directing agent is at least one selected from the tetramethylammonium salts, tetraethylammonium salts, tetrapropylammonium salts, tetrabutylammonium salts and other quaternary alkylammonium salts and amines.
The present invention comprises the following technical means:
(9) A zeolite characterized by having the chemical composition represented by [(Si₃₆₋ₓT_{y}·O₇₂) ·M_{z}] (wherein M is a cation of an alkali metal such as Li, Na, K or Rb, T represents Al, Ga or Fe as skeleton substituting elements, x satisfies 0 ≤ x ≤ 3.0, y satisfies 0 ≤ y ≤ 1.0 and z satisfies 0 ≤ z ≤ 3.0), and having a micropore structure made up of covalent bonds between Si and O atoms.
(10) The zeolite according to (9) above, wherein the lattice spacing d (Å) in the powder x-ray diffraction pattern is as described in Tables 8 and 9 below.

**Table 8**

| d(Å) | Relative strength |
|---|---|
| 9.17±0.05 | 100 |
| 6.86±0.05 | 35 |
| 6.11±0.05 | 5 |
| 5.50±0.05 | 4 |
| 4.84±0.05 | 1 |
| 4.70±0.05 | 1 |
| 4.58±0.05 | 3 |
| 4.44±0.05 | 7 |
| 4.35±0.05 | 7 |
| 4.09±0.05 | 6 |
| 3.88±0.05 | 8 |
| 3.81±0.05 | 9 |
| 3.68±0.05 | 3 |
| 3.43±0.05 | 25 |
| 3.41±0.05 | 29 |
| 3.31±0.05 | 8 |
| 3.24±0.05 | 9 |
| 3.07±0.05 | 1 |

**Table 9**

| d(Å) | Relative strength |
|---|---|
| 9.25±0.05 | 100 |
| 8.85±0.05 | 7 |
| 7.67±0.05 | 4 |
| 6.85±0.05 | 65 |
| 6.14±0.05 | 7 |
| 4.74±0.05 | 6 |
| 4.65±0.05 | 7 |
| 4.49±0.05 | 13 |
| 4.40±0.05 | 5 |
| 4.10±0.05 | 5 |
| 3.90±0.05 | 7 |
| 3.84±0.05 | 8 |
| 3.71±0.05 | 5 |
| 3.44±0.05 | 30 |
| 3.34±0.05 | 14 |
| 3.26±0.05 | 9 |
| 3.08±0.05 | 4 |
| 2.99±0.05 | 3 |
| 2.89±0.05 | 2 |
| 2.75±0.05 | 1 |
| 2.37±0.05 | 2 |
| 1.97±0.05 | 2 |
| 1.86±0.05 | 2 |

(11) The zeolite according to (9) above, wherein the crystal structures can be described as orthorhombic with crystal lattice constants in the range of a = 18.35 ± 0.05 Å, b = 13.77 ± 0.03, c = 7.37 ± 0.03 Å (space group Pnma), orthorhombic with lattice constants in the range of a = 18.35 ± 0.05 Å, b = 13.77 ± 0.03, c = 7.37 ± 0.03 Å (space group Pnnm), orthorhombic with lattice constants in the range of a = 18.35 ± 0.05 Å, b = 13.77 ± 0.03, c = 14.74 ± 0.03 Å (space group Pbcm) and monoclinic with lattice constants in the range of a = 18.35 ± 0.05 Å, b = 13.77 ± 0.03, c = 7.37 ± 0.03 Å, β = 90 ± 0.3° (space group P21/m).
(12) The zeolite according to (9) above, wherein the local coordination of the O atoms surrounding the Si atoms in the skelton structure is tetracoordinate.
(13) The zeolite according to (9) above, wherein the skeletal structure formed by the binding of the Si and O atoms has a regular geometry.
(14) The zeolite according to (9) above, having pores with a mean size of 0.48 nm or more due to gas adsorption.
(15) A method for manufacturing a zeolite characterized by performing dehydration polycondensation of the crystalline layered compound defined in (1) above, to synthesize a zeolite with the chemical composition represented by [(Si₃₆₋ₓTy·O₇₂) ·M_{Z}] (wherein M is a cation of an alkali metal such as Li, Na, K or Rb, T represents Al, Ga or Fe as skeleton substituting elements, x satisfies 0 ≤ x ≤ 3.0, y satisfied 0 ≤ y ≤ 1.0 and z satisfies 0 ≤ z ≤ 3.0).
(16) The method for manufacturing a zeolite according to (15) above, wherein manufacture is in a vacuum in the range of 1 x 10⁻³ to 1 x 10⁻⁸ torr as a condition for dehydration polycondensation.
(17) The method for manufacturing a zeolite according to (15) above, wherein the heating temperature for dehydration polycondensation is 400 to 800°C.
(18) The method for manufacturing a zeolite according to (15) above, wherein the zeolite is manufactured at atmospheric pressure as a condition for dehydration polycondensation.
(19) The method for manufacturing a zeolite according to (15) above, wherein the heating temperature for dehydration polycondensation is 300 to 800°C.
(20) The method for manufacturing a zeolite according to (15) above, wherein the rate of temperature rise is 0.5 to 50°C per minute.
(21) The method for manufacturing a zeolite according to (15) above, wherein as a combustion-supporting gas a gas comprising oxygen molecules in a molecular state is used.
(22) A catalyst or separation/adsorption material comprising the zeolite according to any of (9) to (14) above.

The present invention is explained below with reference to the drawings beginning with the crystalline layered compound and ending with the zeolite.

To give a general explanation of this crystalline layered compound, the layered compound shown in Figures 2 and 3 has a basic silicate structure of repeating units of Si-O tetrahedral coordination, with micropores formed by silicon 5-member rings included in the silicate. Layers of a cation with an ion radius of 1.0 angstrom or more or an organic structure directing agent such as an amine with a diameter of 3.0 angstroms or more are also included between the silicate layers to form the whole.

For the crystalline layered compound which is the raw material in the method of the present invention, a layered silicate containing an organic structure directing agent is synthesized from a silica source, an alkali source with an ion radius of 1.0 angstroms or more, an amine or other organic structure directing agent with a diameter of 3.0 angstroms or more and a solvent. SiO₂ for example is preferably used as the silica source, but this is not a limitation.

Any known organic structure directing agent can be used as long as it can intervene between layers and has the ability to spread the layers or act as a mold in forming the skeletal silicate structure. Examples include tetramethylammonium salts, tetraethylammonium salts, tetrapropylammonium salts, tetrabutylammonium salts and other quaternary alkyl ammonium salts as well as amines and phosphonium ions (R₄P⁺, where R is hydrogen, an alkyl with 10 or fewer carbon atoms or an aryl). A tetramethylammonium salt can be used by preference as the organic structure directing agent in the present invention.

The reaction components and molar ratios thereof in this manufacturing method can be determined appropriately according to the composition of the target crystalline layered compound represented by the formula above.

The process for reacting the crystalline layered compound from a sol-gel mixed solution produced by mixing the raw materials comprises heating an autoclave or other reaction container when heat treatment is carried out in the presence of large quantities of water. The reaction temperature is not particularly limited but is preferably 100 to 200°C or more preferably 140 to 170°C, while the reaction time is preferably 3 hours to 30 days or more preferably 3 days to 14 days. This resulting powder is washed with acetone and water and dried.

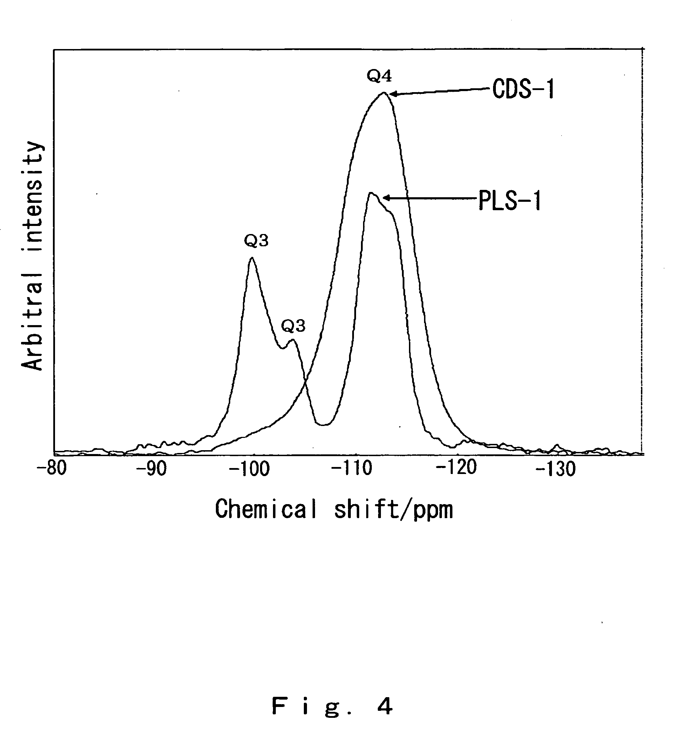

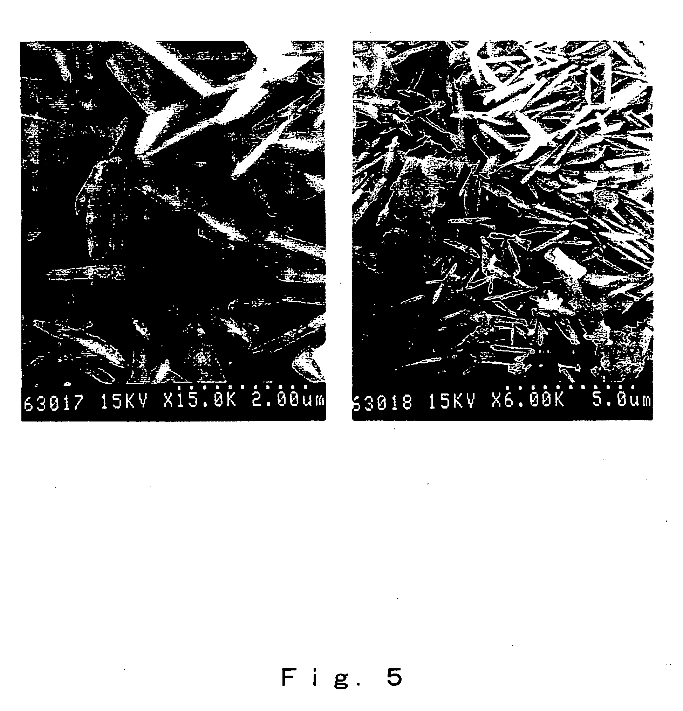

²⁹Si-MAS NMR, SEM observation and powder XRD were used to confirm that the resulting compound was layered. The following analysis data were obtained using the samples of Example 1 below. The coordination of the O atoms around the Si as analyzed by ²⁹Si-MAS NMR is shown in Figure 4. Peaks assigned to Q³ near -98 ppm to -104 ppm and Q⁴ near -113 ppm were observed in the spectrum. Q³ indicates the presence of 3 Si-O groups around each Si, while Q⁴ indicates the presence of 4 Si-O groups. Because Si is tetrafunctional, the remaining functional group of Q³ is not Si-O. In this case, O becomes O⁻ or OH. Normally a zeolite has a completely closed Si-O network, meaning that only Q⁴ appears. The inclusion of Q³ means that the network is partially broken and the silicate has a layered structure. The presence of silicon 5-membered rings was shown by powder XRD structure analysis, which yielded a crystal structure similar to the conceptual structure shown in Figures 2 and 3. SEM photography revealed scale-shaped crystals 1 to 2 mm on a side and about 0.2 mm thick, as shown in Figure 5.

Moreover, crystalline layered compound PLS-1 has a crystal structure exhibiting the characteristic diffraction peaks shown in Table 10 below in powder x-ray diffraction:

**Table 10**

| d (Å) | Relative strength |
|---|---|
| 10.47±0.2 | vs |
| 8.38±0.15 | w |
| 7.34±0.15 | m |
| 7.00±0.1 | m |
| 6.51±0.1 | m |
| 6.45±0.1 | s |
| 5.86±0.05 | m |
| 5.82±0.04 | m |
| 5.66±0.04 | w |
| 5.23±0.04 | m |
| 5.07±0.04 | w |
| 4.90±0.04 | s |
| 4.75±0.04 | m |
| 4.57±0.04 | w |
| 4.40±0.04 | m |
| 4.35±0.04 | s |
| 4.26±0.04 | s |
| 4.19±0.04 | vs |
| 4.00±0.04 | m |
| 3.94±0.035 | s |
| 3.85±0.035 | s |
| 3.83±0.035 | vs |
| 3.78±0.035 | w |
| 3.67±0.035 | m |
| 3.63±0.035 | s |
| 3.60±0.035 | w |
| 3.55±0.035 | m |
| 3.51±0.035 | m |
| 3.50±0.035 | vs |
| 3.48±0.035 | vs |
| 3.38±0.035 | m |
| 3.34±0.035 | w |
| 3.32±0.035 | s |

(wherein d is the lattice spacing, w = weak relative strength, m = moderate relative strength, s = strong relative strength, vs = very strong relative strength).

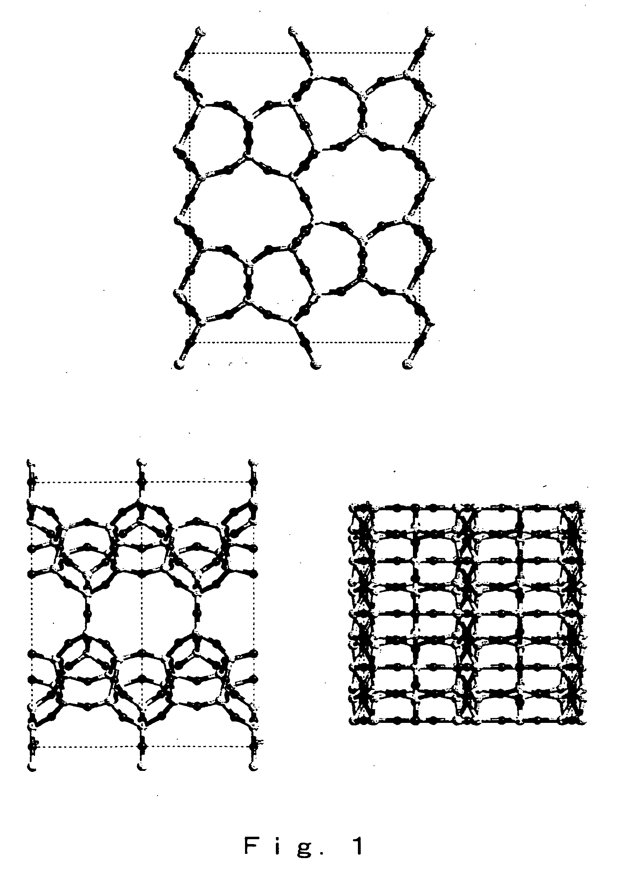

To give a general explanation of the CDS-1 zeolite, it is a high-silica-content zeolite having a basic silicate structure of repeating units of tetrahedral Si-O coordination, and having a crystal structure wherein pores comprising silicon 5-member and 8-members rings make up the whole in the atomic arrangement shown in Figure 1.

CDS-1 zeolite has a crystal structure exhibiting the characteristic diffraction peaks shown in Table 11 below in powder x-ray diffraction.

**Table 11**

| d (Å) |
|---|
| 9.17±0.05 |
| 6.86±0.05 |
| 6.11±0.05 |
| 5.50±0.05 |
| 4.58±0.05 |
| 4.44±0.05 |
| 4.35±0.05 |
| 4.09±0.05 |
| 3.88±0.05 |
| 3.81±0.05 |
| 3.68±0.05 |
| 3.43±0.05 |
| 3.41±0.05 |
| 3.31±0.05 |
| 3.24±0.05 |

CDS-1 zeolite has a structure in which cylindrical pores of different sizes overlap each other on a sheet as shown in Figure 1, and the crystal structure is liable to strain due to layering irregularities and the like in the sheet skeleton. In some cases, it has a crystal structure exhibiting the characteristic diffraction peaks shown in Table 12 below in addition to the aforementioned diffraction pattern.

**Table 12**

| d (Å) |
|---|
| 9.25±0.05 |
| 8.85±0.05 |
| 7.67±0.05 |
| 6.85±0.05 |
| 6.14±0.05 |
| 4.74±0.05 |
| 4.65±0.05 |
| 4.49±0.05 |
| 4.40±0.05 |
| 4.10±0.05 |
| 3.90±0.05 |
| 3.84±0.05 |
| 3.71±0.05 |
| 3.44±0.05 |
| 3.34±0.05 |
| 3.26±0.05 |
| 3.08±0.05 |

Even in this case, the skeletal topology is basically identical to that of the CDS-1 zeolite shown in Table 11 according to the ²⁹Si-MAS NMR and nitrogen adsorption measurement results below. Consequently, although the average structure may be a crystal structure which is somewhat distorted or lacking in symmetry, the geometric relationships of the skeleton remain as shown in Figure 1.

CDS-1 zeolite can be obtained by heating PLS-1 either in vacuum or in a flow of combustion-supporting gas, and the optimal heating conditions are different in each case. The vacuum synthesis method is explained first. PLS-1 powder crystals obtained as discussed above are placed alone in a Pyrex™ or quartz glass tube, connected to a vacuum line equipped with a nitrogen trap, and heat treated in vacuum to obtain CDS-1 zeolite by dehydration polycondensation. In this case the ultimate vacuum is not particularly limited but is preferably in the range of 1 × 10⁻³ to 1 × 10⁻⁸ torr. The heating temperature is also not particularly limited but is preferably 400 to 800°C. The degree of vacuum falls during the process of dehydration polycondensation, and then rises again when the transition to zeolite is complete.

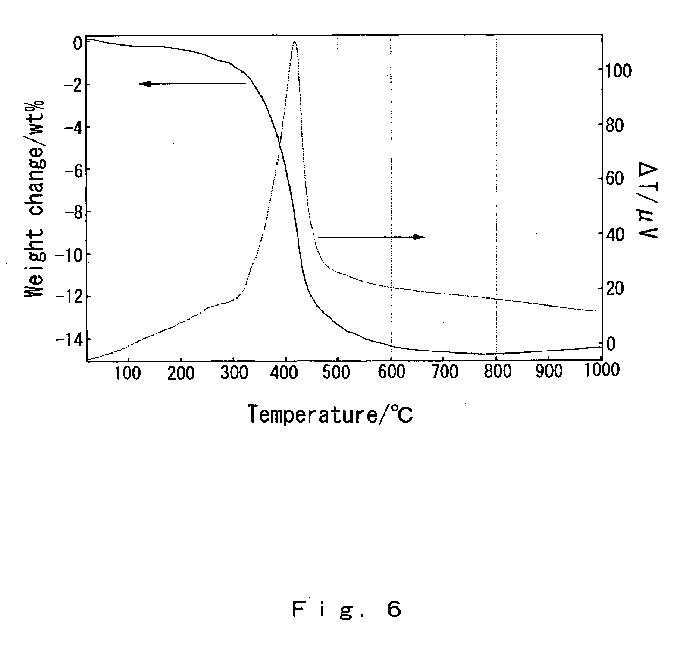

A compound obtained by heat treatment in vacuum has about 15% less than its original weight (Figure 6). This means that the organic structure directing agent contained in the crystalline layered PLS-1 compound has been combusted or released, and almost none is included as molecules in the crystal structure of the CDS-1 zeolite product. The resulting powder product is gray, and includes a carbide residue.

When the residue needs to be removed, for example 1000 mg of CDS-1 zeolite crystals are placed in an alumina Petri dish, heated at 1.4°C/min from room temperature to 650°C in a 1000 ml/min air flow using a muffle furnace, and maintained at 650°C for 4 hours. The final product is a white powder. Powder XRD measurement of the zeolite of this example reveals the characteristics diffraction peaks of CDS-1 zeolite shown in Table 11.

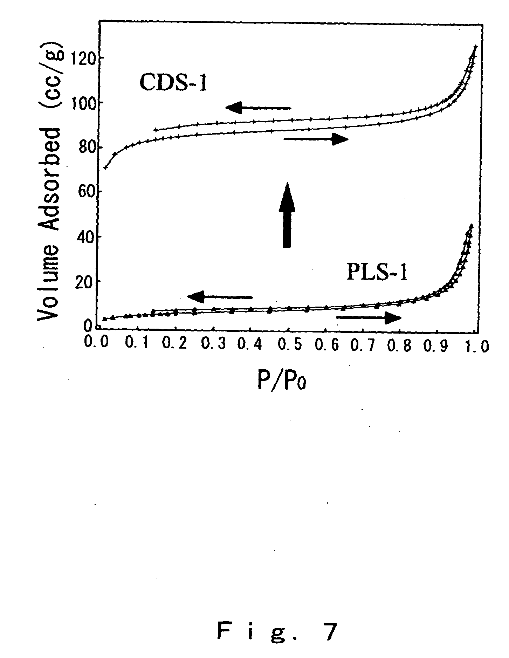

Judging by the nitrogen gas adsorption isotherm curves for CDS-1 zeolite and the layered compound PLS-1 shown in Figure 7, CDS-1 has a much greater adsorption volume, indicating that it has changed to a zeolite compound with a pore structure and a larger adsorption surface area.

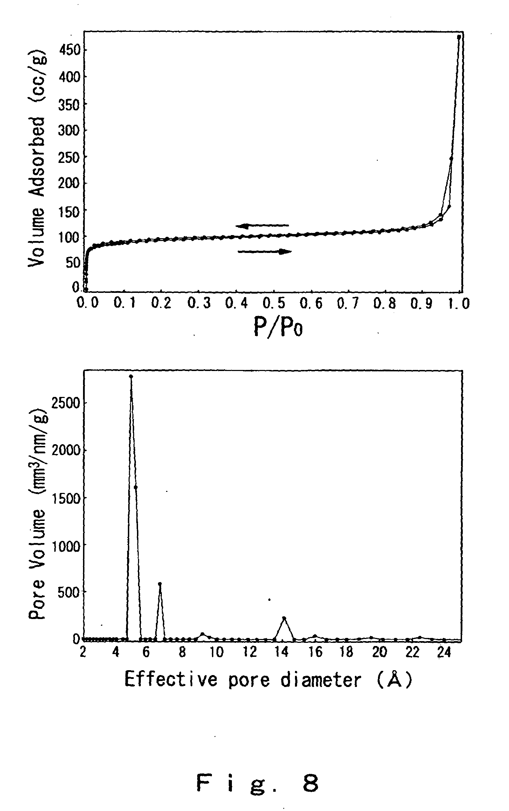

The results for pore size distribution of the zeolite as analyzed by the density half-function method from the argon gas adsorption isotherm curve are shown in Figure 8. The zeolite of this example has a high gas adsorption capacity and a pore size distribution of 0.48 nm or more, showing that it has micropores of roughly the same size as the pores of known zeolites. The total pore volume is 0.6 ml/g.

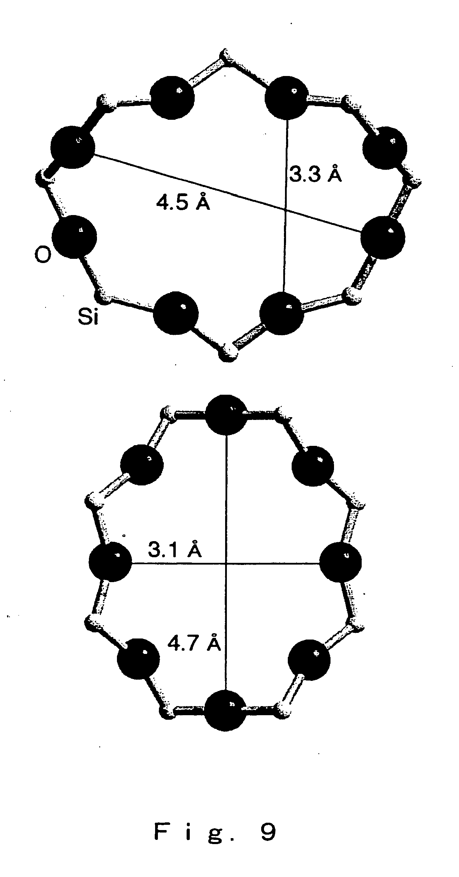

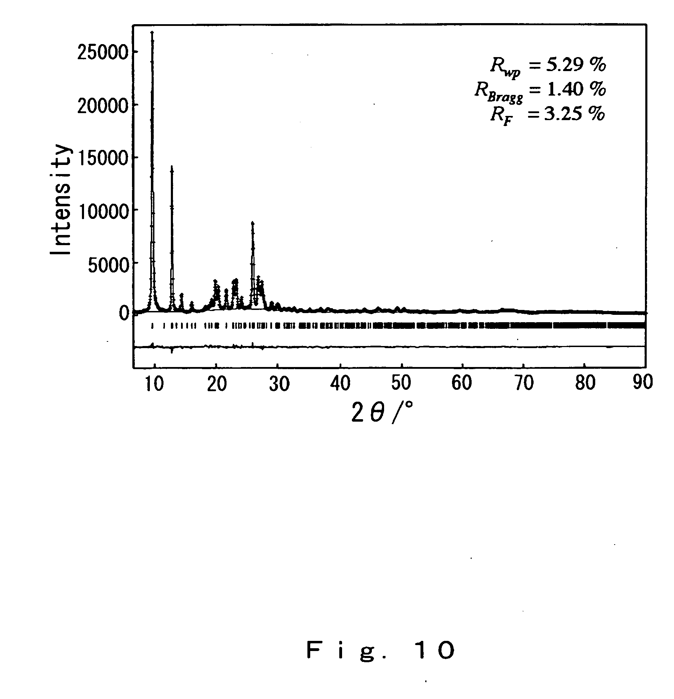

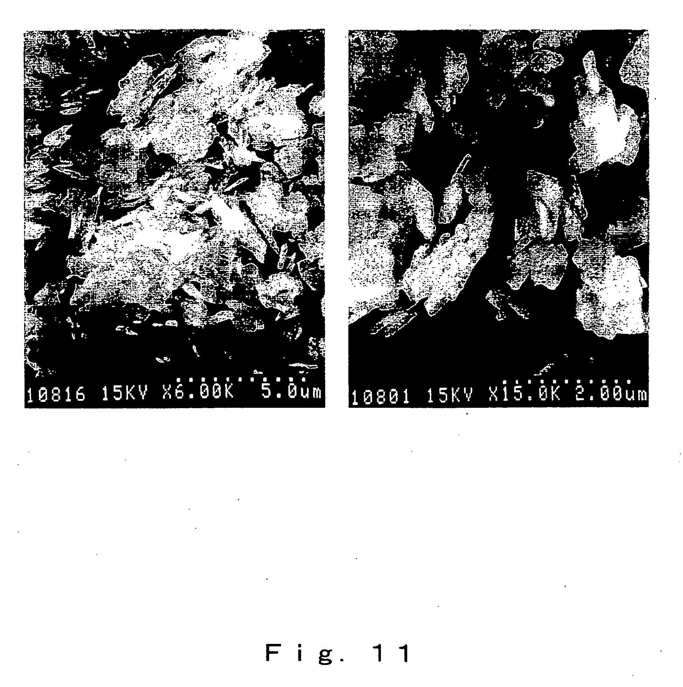

Proof that the compound created in this way is a zeolite having a pore structure is provided by ²⁹Si-MAS NMR, Ar gas adsorption results and detailed crystal structure analysis using the powder XRD data. These analysis data were obtained by analyzing the samples from Example 1 below. The ²⁹Si-MAS NMR spectrum is shown in Figure 4. Only a peak assigned to Q⁴ appears in the spectrum. Because zeolites normally have a Si-O network structure which is completely closed apart from the external crystal surfaces, statistically only a Q⁴ signal is observed. This is evidence that the local structure derives from a pore structure characteristic of zeolites. The presence of pores formed by silicon 5-member and 8-member rings is confirmed by the fact that based on crystal structure analysis using the powder XRD data, the crystal structure is extremely similar to that shown in Figure 1. There are two kinds of newly-formed silicon 8-member rings, estimated to have the effective sizes shown in Figure 9. When the structural model of Figure 1 was subjected to a more precise Rietveld analysis, a satisfactory reliability factor was obtained as shown in Figure 10. SEM photography revealed scale-shaped crystals 1 to 2 mm on a side and about 0.2 mm thick as shown in Figure 11, not much different from the morphology of the layered compound PLS-1.

CDS-1 can be obtained even in a flow of combustion-supporting gas if the conditions are optimized. The same PLS-1 powder crystals used above are placed in a Pyrex™ or quartz tube, and heated in a flow of combustion-supporting gas to obtain CDS-1 by dehydration polycondensation. The flow rate of combustion-supporting gas is not particularly limited but should preferably be 10 mL or more per minute per 50 mg of PLS-1. More preferably the flow rate is about 10 to 100 mL per minute per 50 mg of PLS-1.

A combustion-supporting gas is a gas comprising oxygen, and is a gas comprising oxygen molecules such as pure oxygen or air. Dry air was used here as the combustion-supporting gas. As in ordinary zeolite synthesis, this is necessary for effective combustion of the residual organic template.

The first aspect of the present invention is explained below.

The inventors in this case discovered that a zeolite having a crystal structure comprising silicon 5-member and 8-member ring structures could be synthesized from a layered silicate (PLS) having a crystal structure comprising silicon 5-member rings by exploiting their geometric similarity. Specifically, a novel zeolite was produced by condensing the Si-OH groups in PLS under conditions such as vacuum, air or oxygen atmosphere. This zeolite CDS-1 is a porous substance (zeolite) with a geometric crystal structure (atomic arrangement) having the chemical composition represented by [(Si₃₆₋ₓ· O₇₂). M_{y}] (wherein M is a cation of an alkali metal such as Na, K Li or Rb, x satisfies 0 ≤ x ≤ 3.0, y satisfied 0 ≤ y ≤ 3.0), and a pore structure comprising covalent bonds between Si and O atoms. The pore size of CDS-1 is small for a zeolite, and it could potentially be used as a separation membrane for low-molecular-weight gases such as carbon dioxide (CO₂), methane (CH₄), ethane (C₂H₆), propane (C₃H₈) and the like. Moreover, in the CDS-1 of the present invention the lattice spacing d (Å) in the x-ray diffraction pattern is at least as described in Tables 4 and 5.

Next, preferred embodiments of the CDS-1 zeolite membrane and manufacturing method therefor of the present invention are explained. In the present invention descriptions of numerical ranges include not only the outside values but also all intermediate values, and appropriate design changes, improvements and the like can be added based on ordinary knowledge to the extent that they do not deviate from the intent of the present invention.

Methods of synthesizing the novel high-silica zeolite CDS-1 membrane of the present invention include for example (1) a method of forming its precursor substance PLS on a porous support and condensing the Si-OH groups in the PLS by baking and/or vacuum treatment to convert it to a CDS-1 membrane and (2) a method of applying CDS-1 powder to a porous support by implantation, suction or the like, and then performing hydrothermal synthesis in an aqueous solution comprising alkali and silica sources to form a membrane by two-dimensional growth of the CDS-1 crystals. In these methods, a substance identical to the crystalline layered compound PLS (Japanese Patent Application 2002-331333) is used as the precursor compound in synthesizing the CDS-1 zeolite membrane.

The support in the present invention may be a porous support, anodic oxidation coating porous support or the like made of a metal, alloy or metal oxide such as alumina, mullite, zirconia, stainless steel or aluminum. Preferably the porous support has a mean pore size of 0.1 to 10 microns, and examples include PM tubes (tubular supports) and F (flat disc or square plate) and other products (Nikkato Co.). Methods of surface treating these supports include water washing, ultrasound cleaning and the like, but preferably the surface of the support is ultrasound cleaned for 1 to 10 minutes using water.

In the present invention the PLS membrane is formed on the porous support by hydrothermal synthesis. PLS has a basic silicate structure of repeating units of SiO tetrahedral coordination, with micropores formed by silicon 5-member rings inside the silicate. It is also a layered silicate containing an organic structure directing agent which is synthesized from a silica source, an alkali source with an ion radius of 1.0 Å or more, an amine or other organic structure directing agent with a diameter of 3.0 angstroms or more and a solvent. The membrane can be prepared on a support for example by first rubbing previously-synthesized PLS seed crystals onto a porous support, and then repeating hydrothermal synthesis to form a strong, continuous membrane by growing the seed crystals. An appropriate container such as a pressure container can be used for this hydrothermal synthesis. The porous support can be arranged inside the pressure container either on the bottom, middle or top of the container, and may be parallel, perpendicular, or at a particular angle to the container. The PLS crystallization speed can be accelerated by including 0.1 to 30 wt% or preferably 3 to 10 wt% of the PLS seed crystals in the starting raw materials during hydrothermal synthesis.

In the present invention, in the process of CDS-1 zeolite synthesis a PLS membrane is first obtained by forming a membrane of the aforementioned crystalline layered compound PLS as the precursor compound on the aforementioned porous support. Next, the resulting PLS membrane is baked in air at 300 to 800°C or preferably 400 to 600°C at a rate of 0.1 to 30°C or preferably 0.5 to 10°C per minute to convert it to a CDS-1 membrane. The purpose of this baking process is to condense the Si-OH groups of the PLS, and conversion to a CDS-1 membrane can also be accomplished by baking in a vacuum, such as for example by placing the PLS membrane in a glass tube of any size, connecting the glass tube to general-use vacuum line equipped with a nitrogen trap and turbo molecular pump, and heat treating in a vacuum. In this case, the ultimate vacuum is not particularly limited but is preferably 1 × 10⁻³ to 1 × 10⁻⁸ torr, while the heating temperature is preferably 400 to 800°C.

In the CDS-1 constituting the zeolite of the present invention the crystal structures can be described as (1) orthorhombic with crystal lattice constants in the range of a = 18.35 ± 0.05 Å, b = 13.77 ± 0.03, c = 7.37 ± 0.03Å (space group Pnma), (2) orthorhombic with lattice constants in the range of a = 18.35 ± 0.05 Å, b = 13.77 ± 0.03, c = 7.37 ± 0.03 Å (space group Pnnm), (3) orthorhombic with lattice constants in the range of a = 18.35 ± 0.05 Å, b = 13.77 ± 0.03, c = 14.74 ± 0.03Å (space group Pbcm) and (4) monoclinic with lattice constants in the range of a = 18.35 ± 0.05Å, b = 13.77 ± 0.03, c = 7.37 ± 0.03Å, β = 90 ± 0.3° (P21/m). CDS-1 is also characterized by a lattice spacing d (Å) in the x-ray diffraction pattern which is at least as shown in Tables 4 and 5. Another example of a method for manufacturing a CDS-1 membrane is a method of coating a porous support with seed crystals of previous-synthesized CDS-1, and then forming a membrane by secondary crystal growth. In this method, the PLS is synthesized and then the Si-OH groups in the PLS are condensed to form CDS-1. Next, a porous support is coated with CDS-1 seed crystals, and secondary growth of the seed crystals is accomplished in an alkali aqueous solution or the like comprising silicon to form the CDS-1 membrane. Because the resulting CDS-1 membrane not only exhibits catalytic activity in vapor-phase Beckmann rearrangement reactions for example but also has an Al-SiO₂ 8-member ring structure (4.5 × 3.3 Å), it is useful as a heat resistant, acid-resistant member for use as a separation membrane material. For example, when water-ethanol separation was performed by the osmoticvaporation method using this CDS-1 membrane, the separation factor of water from ethanol was 23, and the permeation flow rate was 0.23 kg/m²·h. It is believed to be the presence of residual Si-OH remaining in the membrane after condensation from PLS which gives the CDS-1 the properties of a selective permeation membrane.

Next, the second aspect of the present invention is explained.

The present invention relates to the manufacture of ε-caprolactam by Beckmann rearrangement from cyclohexanone oxime using as the catalyst a zeolite (CDS-1) obtained by dehydration polycondensation of a crystalline layered silicate compound and having the chemical composition represented by [(Si₃₆₋ₓT_{y}·O₇₂) ·M_{z}] (wherein M is a cation of an alkali metal such as Li, Na, K or Rb, T represents Al, Ga, Fe and Ce as skeleton substituting elements, x satisfies 0 ≤ x ≤ 3.0, y satisfied 0 ≤ y ≤ 1.0 and z satisfies 0 ≤ z ≤ 3.0), a micropore structure formed by of covalent bonds between Si and O atoms, and a geometric crystal structure (atomic arrangement) comprising silicon 5-member and 8-member rings.

Regarding the structural properties of the CDS-1 zeolite used in the method of the present invention, this CDS-1 is a high-silica-content zeolite having a basic silicate structure of repeating units of Si-O tetrahedral coordination, and having a crystal structure (atomic arrangement) composed overall of a geometric arrangement of pores formed by 5-member and 8-member silicon rings. The zeolite used in the present invention can be obtained by any method as long as it has these structural characteristics, with no particular limits on the manufacturing method.

The CDS-1 zeolite used in the present invention has a crystal structure exhibiting the characteristic diffraction peaks shown in Table 13 below in powder x-ray diffraction.

**Table 13**

| d(Å) |
|---|
| 9.17±0.05 |
| 6.86±0.05 |
| 6.11±0.05 |
| 5.50±0.05 |
| 4.84±0.05 |
| 4.70±0.05 |
| 4.58±0.05 |
| 4.44±0.05 |
| 4.35±0.05 |
| 4.09±0.05 |
| 3.88±0.05 |
| 3.81±0.05 |
| 3.68±0.05 |
| 3.43±0.05 |
| 3.41±0.05 |
| 3.31±0.05 |
| 3.24±0.05 |
| 3.07±0.05 |

Moreover, the CDS-1 zeolite of the present invention has a structure in which cylindrical pores of different sizes overlap each other on a sheet, and the crystal structure is liable strain due to layering irregularities and the like in the sheet skeleton. In some cases, it has a crystal structure exhibiting the characteristic diffraction peaks shown in Table 14 below in addition to the aforementioned diffraction pattern.

**Table 14**

| d(Å) |
|---|
| 9.25±0.05 |
| 8.85±0.05 |
| 7.67±0.05 |
| 6.85±0.05 |
| 6.14±0.05 |
| 4.74±0.05 |
| 4.65±0.05 |
| 4.49±0.05 |
| 4.40±0.05 |
| 4.10±0.05 |
| 3.90±0.05 |
| 3.84±0.05 |
| 3.71±0.05 |
| 3.44±0.05 |
| 3.34±0.05 |
| 3.26±0.05 |
| 3.08±0.05 |

Even in this case, the pore structure is basically identical to that of CDS-1 zeolite according to the ²⁹Si-MAS NMR and nitrogen adsorption measurement results below. Consequently, although the average structure of CDS-1 zeolite may deviate from an orthorhombic crystal structure in the direction of a somewhat distorted crystal structure with low symmetry, the geometric relationships of the skeleton remain as shown in Figure 1.

Next, an example of a method for synthesizing the high-silica zeolite catalyst CDS-1 is explained.

In the present invention, the precursor compound for synthesizing the CDS-1 zeolite may preferably be identical to the crystalline layered compound PLS-1 (Japanese Patent Application No. 2002-331333) for example. To give a detailed explanation of this PLS-1 (Pentasil Llayered Silicate), this crystalline layered compound has a basic silicate structure of repeating units of Si-O tetrahedral coordination, with micropores formed by silicon 5-member rings included inside the silicate. This PLS-1 is preferably synthesized as a layered silicate containing an organic structure directing agent from for example a silica source, an alkali source with an ion radius of 1.0 angstroms or more, an amine or other organic structure directing agent with a diameter of 3.0 angstroms or more and a solvent. The quaternary amine TMAOH (tetramethylammonium hydroxylate) for example can be used favorably as the structure directing agent.

The reaction components and molar ratios thereof in this PLS-1 manufacturing method are determined appropriately according to the composition of the crystalline layered compound represented by [(Si₁₈₋ₓ·O₃₈) ·M_{y}·(TMA)_{z} · (H₂O)_{w}] (wherein TMA is a tetraalkylammonium cation, M is a cation of an alkali metal such as Na, K or Li, x satisfies 0 ≤ x ≤ 1.2, y satisfies 0.5 ≤ y ≤ 1.5, z satisfies 6 ≤ z ≤ 8 and w satisfies 0.02 ≤ w ≤ 1.5).

The resulting PLS-1 is placed in a glass tube of any size which is then connected to a general-use vacuum line equipped with a nitrogen trap and turbo molecular pump, and CDS-1 zeolite is obtained by heat treatment in vacuum. In this case, the ultimate vacuum is preferably in the range of 1 × 10⁻³ to 1 × 10⁻⁸ torr, while the heating temperature is preferably 300 to 800°C or more preferably 400 to 800°C.

The compound obtained by the aforementioned heat treatment has about 20% less than its original weight. The final product is a white powder exhibiting diffraction peaks identical to those shown in Table 2 in powder XDR analysis, which are the characteristic peaks of CDS-1 zeolite.

This zeolite has high gas adsorption ability. This zeolite also has exterior surface adsorption properties because it has a history of nitrogen gas adsorption-desorption. The effective pore sizes of the two types of silicon 8-member rings included in the Si-O skeletal structure as calculated based on the crystal structure model are shown in Figure 9. The radius of an oxygen atom is given as 1.35Å based on the literature (Ch. Baerlocher, W. M. Meier and D. H. Olson, 2001, p. 11, Atlas of Zeolite Framework Types, Elsevier). As shown in Figure 9, the average pore size is 0.48 nm, which is similar to the pore sizes of known zeolites.

It was confirmed based on the ²⁹Si-MAS NMR, SEM and powder XRD measurements and on crystal structure analysis that the compound synthesized in this way was a zeolite having a pore structure. Only a peak assigned to Q⁴ is seen in the ²⁹Si-MAS NMR spectrum. Normally a zeolite has a completely closed Si-O network, meaning that only Q⁴ appears. This is evidence that the local structure is derived from a pore structure characteristic of zeolites. The presence of pores formed by silicon 5-member and 8-member rings was confirmed by the fact that based on crystal structure analysis using the powder XRD data, the crystal structure is extremely similar to that shown in Figure 1.

Thus, the novel high-silica zeolite catalyst CDS-1 used in the present invention is synthesized by heating the pentasil layered compound PLS under reduced pressure to cause dehydration condensation of the silanol groups between the layers, resulting in a cyclic zeolite, and offers the advantages of ease of manufacturing and a uniform resulting zeolite. Moreover, the quaternary amine TMAOH (tetramethylammonium hydroxylate) used as the structure directing agent during PLS synthesis is inexpensive, costing only about 1/2 to 1/3 as much commercially as the expensive TPAOH (tetrapropylammonium hydroxylate) used in MFI silicalite and silicalite. Moreover, since most of the TMAOH used in synthesizing CDS-1 from PLS is recovered, a major advantage is that the TMAOH can be reused. Another advantage is that a baking operation is not required to remove the TMAOH.

In the present invention, a method of dehydration polycondensing the crystalline layered silicate compound in atmosphere is preferably adopted for the process of synthesizing the aforementioned CDS-1. In this case, the heating temperature is preferably 300 to 800°C, while the rate of temperature rise is preferably 0.1 to 10°C per minute. In the present invention, the CDS-1 is preferably CDS-1 obtained by treating a precursor thereof with a group 6 transitional metal oxide (Cr, Mo, W or the like), or CDS-1 having micropores with a mean pore size of 0.48 nm or more based on pore distribution analysis using nitrogen adsorption, with a pore volume of 0.6 cc/g or more, or CDS-1 which has further undergone cation exchange or hydrogen ion exchange, such as CDS-1 which has been ion exchanged and proton substituted with ammonium nitrate, but it is not limited to these.

In the method of the present invention for manufacturing ε-caprolactam from cyclohexanone oxime, the reaction temperature is preferably 150 to 500°C and the cyclohexanone oxime WHSV is preferably between 0.001 h-1 and 20.0 h-1. This method features the use of the novel crystalline CDS-1 zeolite catalyst in a Beckmann rearrangement reaction which converts cyclohexanone oxime into ε-caprolactam in a vapor phase, but is otherwise not particularly limited, and ordinary reaction methods, reaction conditions, reaction equipment and the like can be used. It was found for example that ε-caprolactam is obtained with a 95% conversion rate and 85% selectivity at a reaction temperature of 360°C using methanol as the dilution solvent in the present invention.

### EFFECTS OF THE INVENTION

Because the zeolite of the present invention has a novel crystal structure, is inexpensive and has a high silica content and micropores with a mean size of 0.48 nm or more, it can be applied to solids for metal carrier, separation and adsorption agents, shape-selective solid catalysts, ion exchange agents, chromatography fillers and chemical reaction sites and the like. With the zeolite manufacturing method of the present invention it is possible to easily form CDS-1 zeolite having a novel crystal structure. Moreover, in this manufacturing method a high-level structure is obtained by dehydration polycondensing the skeletal structure of the precursor layered compound as is, thus providing a new tool for structural design of novel zeolites at the atomic level, something which was extremely difficult in the past. It is expected that by expanding this manufacturing method to include chemical modifications which add other elements (Al, Ga, transition metal elements, etc.) to the skeleton, it will be possible to produce novel skeletal element-substituted zeolites which also have high catalytic functions.

The first aspect of the present invention has the effects of (1) providing a high-silica zeolite membrane, (2) providing a method for easily synthesizing a CDS-1 zeolite membrane which is not a known zeolite but an entirely new zeolite, (3) providing a dense zeolite membrane which can adequately provide excellent hydrophobicity and heat resistance, (4) allowing the easy and rapid manufacture of a zeolite membrane which can be used in industrial liquid and gas separation processes and the like, and (5) providing a zeolite membrane suitable as a membrane reactor or the like with both separation and catalytic functions in the petrochemical industry for example.

The second aspect of the present invention has the effects of (1) providing a method for manufacturing ε-caprolactam using a novel crystalline CDS-1 zeolite catalyst, (2) allowing ε-caprolactam to be synthesized efficiently using a novel silica zeolite catalyst which is highly functional in Beckmann rearrangements, (3) establishing a new rearrangement reaction which does not suffer the drawbacks of conventional methods, such as the need to use large quantities of fuming sulfuric acid or to treat large quantities of ammonium sulfate bi-product, (4) providing a novel synthesis process using as the zeolite catalyst CDS-1 synthesized at low cost in an easy process.

### BRIEF DESCRIPTION OF THE DRAWINGS

Figure 1 shows the skeletal structure of the CDS-1 zeolite represented by the general formula of the present invention from three arbitrary directions. The white spheres represent Si atoms and the gray spheres O atoms.
Figure 2 shows the similarity in crystal structures between the CDS-1 zeolite represented by the general formula of the present invention and the crystalline layered silicate PLS-1 which is its precursor. The white spheres represent Si atoms and the gray spheres O atoms.
Figure 3 shows the similarity in crystal structures between the CDS-1 zeolite represented by the general formula of the present invention and the crystalline layered silicate PLS-1 which is its precursor from two different perspectives other than that shown in Figure 1.
Figure 4 is a spectrum graph showing the results of ²⁹Si-MAS NMR of the CDS-1 zeolite obtained in the example.
Figure 5 is a scanning electron microscope (SEM) image of the crystalline layered compound PLS-1 obtained in the example.
Figure 6 is a graph of TG-DTA measurements of the crystalline layered compound PLS-1 represented by the general formula of the present invention. Weight change is shown on the left and specific heat on the right.
Figure 7 shows the nitrogen gas desorption and adsorption isotherms of the crystalline layered compound PLS-1 and CDS-1 zeolite obtained in the example. The desorption isotherm is shown above the adsorption isotherm.
Figure 8 shows the argon gas desorption and adsorption isotherms (top) of the CDS-1 zeolite obtained in the example, together with the pore size distribution (bottom) obtained from NLDFT analysis. The desorption isotherm is shown above the adsorption isotherm.
Figure 9 shows pore size as calculated from the skeletal structure of the CDS-1 zeolite obtained in the example.
Figure 10 shows the results of Rietveld analysis of the powder XRD data for the CDS-1 zeolite obtained in the example. (+) indicates an observed value, a solid line a measured value, a vertical bar the position of a black reflection and the bottom line the difference between the observed value and calculated value.
Figure 11 is a scanning electron microscope (SEM) image of the CDS-1 zeolite obtained in the example.
Figure 12 shows the powder XRD patterns for each set temperature during vacuum heat treatment.
Figure 13 is a powder x-ray crystal structure analysis chart for the CDS-1 zeolite of the example which was obtained by raising the temperature to 400°C at 1°C/minute and baking for 5 hours.
Figure 14 is a powder x-ray crystal structure analysis chart for CDS-1 zeolites obtained by raising the temperature to various temperatures at 1°C/minute and baking for 5 hours at those temperatures. An XRD chart of the precursor layered crystalline compound PLS-1 is shown for comparison at the bottom
Figure 15 is a powder x-ray crystal structure analysis chart for CDS-1 zeolites obtained by raising the temperature to 400°C at various rates and baking for 5 hours.

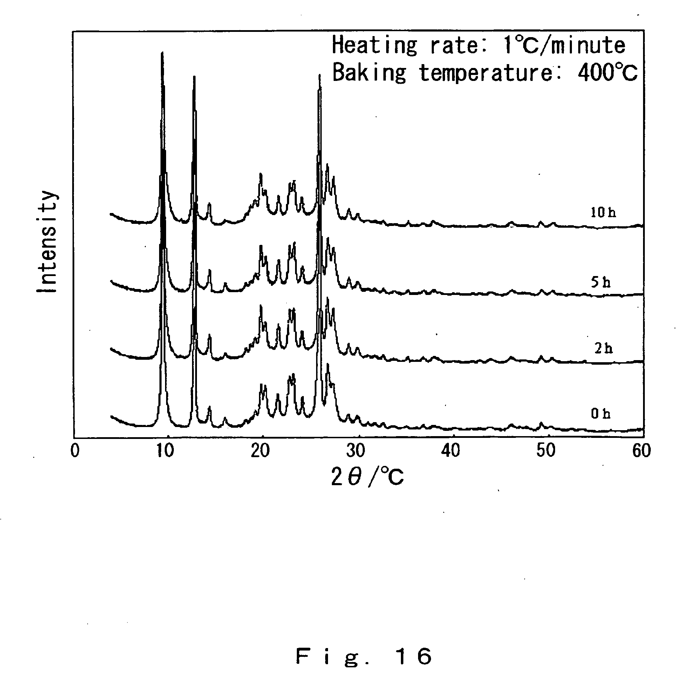

Figure 16 is a powder x-ray crystal structure analysis chart for CDS-1 zeolites obtained by raising the temperature to 400°C at 1°C/minute and baking for various times.

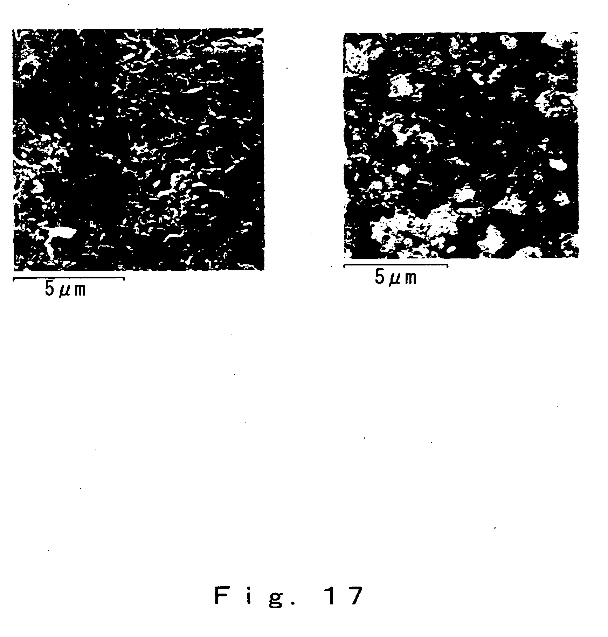

Figure 17 shows electron microscope images of a PLS membrane and a CDS-1 membrane.

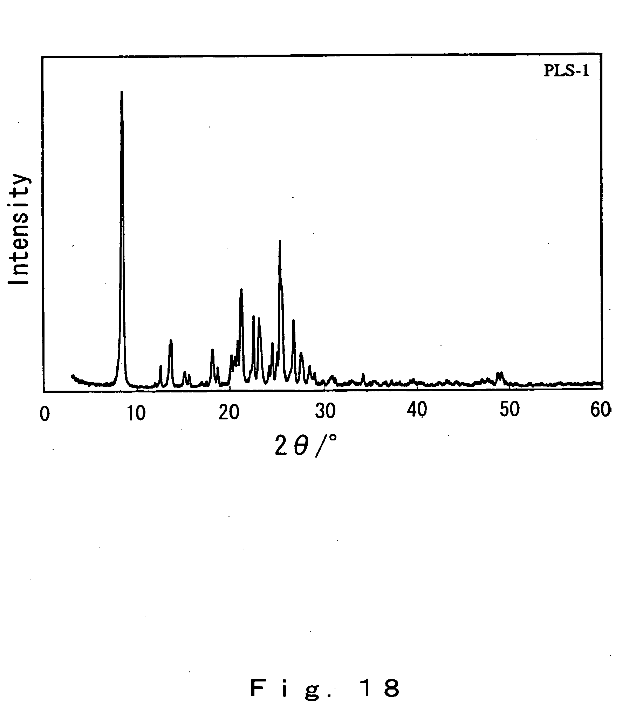

Figure 18 shows a powder x-ray crystal structure analysis chart of PLS.

### BEST MODE FOR CARRYING OUT THE INVENTION

Examples of the aspect outside of the present invention are explained below.

The following powder x-ray diffraction (XRD) patterns were obtained from step scanning at 0.02° intervals using MAC Science M21X and MXP3TA-HR with Cu Ka and Cu Ka₁ beams. The indexing program TREOR90, the Rietveld analysis program RIETAN-2000, the direct method program EXPO (SirWare) and Accelrys Cerius 2 were used. A MAC Science TG-DTA 2000 was used for the thermogravimetric analysis, and a Bruker Biospin AMX-500 for the ²⁹Si-MAS NMR. The argon adsorption isotherm was measured at 81.4 K (liquid argon) using a Quantachrome Autosorb-IMP. The nitrogen adsorption isotherm was measured at 77 K (liquid nitrogen) using a Shimadzu ASAP2010. The chemical composition of the product was determined by ICP analysis (Seiko Instruments SPS-1500R).

### Example 1

### Manufacture of crystalline layered compound PLS-1

10.0 g of SiO₂ (Product name: Cab-O-Sil M5, CABOT Co.) was taken and 22.0 g of 15% TMAOH (tetramethyl ammonium hydroxide: Wako Pure Chemical), 5.0 g of 0.5 standard KOH (Wako Pure Chemical), 25.0 g of H₂O and 50.0 g of 1,4-dioxane (Wako Pure Chemical) were added thereto and agitated well for 1 hour. The mixture was then transferred to an autoclave (content volume 300 ml) having an inner Parr Teflon™ tube, and heat treated for 10 days at 150°C. After being removed from the autoclave, this was washed thoroughly with ethanol and water, and dried for 12 hours at 70°C to obtain a powder product.

This product was confirmed by ²⁹Si-MAS NMR, SEM and XRD measurement to be the layered compound PLS-1. The lattice spacing d (Å) shown in Table 15 was obtained from the powder x-ray diffraction pattern of this product.

**Table 15**

| d(Å) |
|---|
| 10.46±0.10 |
| 8.38±0.05 |
| 7.34±0.05 |
| 7.00±0.05 |
| 6.51±0.05 |
| 6.45±0.05 |
| 5.86±0.05 |
| 5.82±0.05 |
| 5.66±0.05 |
| 5.23±0.05 |
| 5.07±0.05 |
| 4.90±0.05 |

The nitrogen desorption and adsorption isotherms of PLS-1 are shown in Figure 7. PLS-1 is shown to have a low adsorption capacity. The desorption isotherm is shown above the adsorption isotherm. An electron microscope image of PLS-1 is shown in Figure 5. The crystals are scale-shaped and 1 to 2 mm thick, with the characteristics cleavage planes of a layered structure.

### Example 2

Manufacture of crystalline layered compound

The conditions were the same as those used for synthesizing PLS-1 in Example 1, except that heat treatment was for 5 days at 160°C. PLS-1 was obtained as the product in this example.

### Example 3

### Manufacture of crystalline layered compound

The conditions were the same as those used for synthesizing PLS-1 in Example 1, except that heat treatment was for 10 days at 160°C. PLS-1 was obtained as the product in this example.

### Example 4

### Manufacture of crystalline layered compound.

The conditions were the same as those used for synthesizing PLS-1 in Example 1, except that heat treatment was for 5 days at 170°C. PLS-1 was obtained as the product in this example.

### Example 5

### Manufacture of crystalline layered compound

The conditions were the same as those used for synthesizing PLS-1 in Example 1, except that heat treatment was for 10 days at 170°C. In this example some PLS-1 and amorphous silica was produced rather than a single phase of PLS-1. It is thought that a PLS-1 single phase was not produced because heat treatment progressed to far, resulting in conversion to a dense amorphous phase.

### Example 6

### CDS-1 zeolite manufacture 1: heat treatment in vacuum

3.0 g of this PLS-1 was placed in a Pyrex™ or quartz glass tube with an inner diameter of 25 mm which was then attached to a vacuum line, and subjected to a 3-step heat treatment in which the temperature was raised over 4 hours from room temperature to 500°C under 5 × 10⁻⁶ vacuum, maintained for 4 hours, and cooled to room temperature over the course of an hour, to obtain a CDS-1 zeolite product as a gray powder. The weight was 2.55 g. The diffraction pattern shown in Table 16, which is characteristic of CDS-1, was observed in the powder XRD pattern of this product.

**Table 16**

| d(Å) | Relative strength |
|---|---|
| 10.46±0.10 | 100 |
| 7.34±0.05 | 3 |
| 7.00±0.05 | 6 |
| 6.51±0.05 | 8 |
| 6.45±0.05 | 13 |
| 5.86±0.05 | 5 |
| 5.66±0.05 | 5 |
| 5.23±0.05 | 1 |
| 5.07±0.05 | 4 |
| 4.90±0.05 | 13 |
| 4.75±0.05 | 5 |
| 4.40±0.05 | 5 |
| 4.35±0.05 | 14 |
| 4.26±0.05 | 10 |
| 4.19±0.05 | 33 |
| 4.00±0.05 | 4 |
| 3.94±0.05 | 15 |
| 3.85±0.05 | 12 |
| 3.83±0.05 | 20 |
| 3.67±0.05 | 4 |
| 3.62±0.05 | 13 |
| 3.55±0.05 | 6 |

Figure 11 shows a scanning electron microscope image of CDS-1 zeolite. The crystals are scale-shaped crystals 1 to 2 µm on a side and about 0.2 µm thick, and since the crystal morphologies of PLS-1 and CDS-1 are virtually identical, the CDS-1 zeolite was produced by topotactic structural changes from the layered structure PLS-1.

### Example 7

### CDS-1 zeolite manufacture 1: heat treatment in vacuum

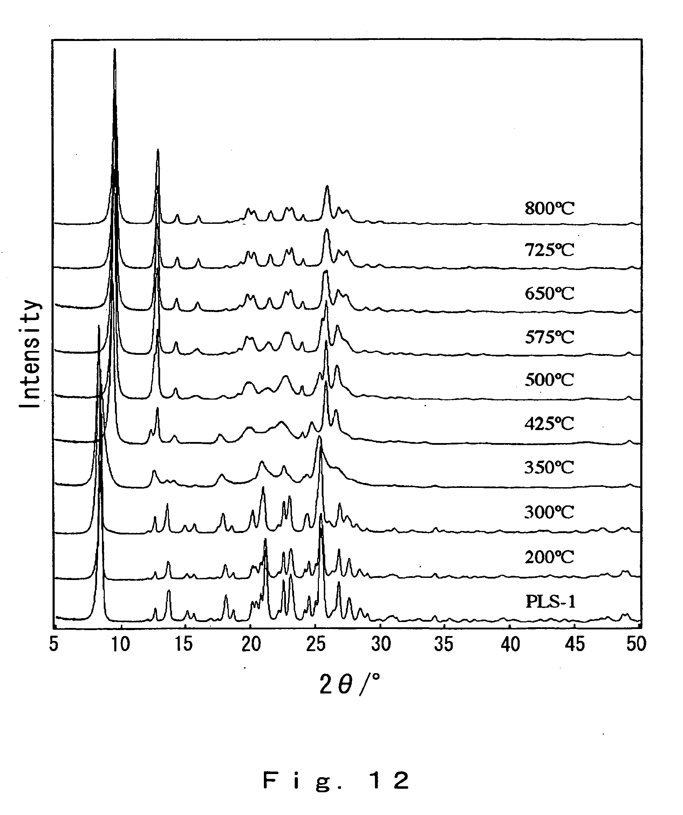

The conditions for synthesizing CDS-1 were the same as those used in Example 1, except that heat treatment was at a set temperature of 575°C. CDS-1 was obtained a the product in this example (Figure 12).

### Example 8

### CDS-1 zeolite manufacture 1: heat treatment in vacuum

The conditions for synthesizing CDS-1 were the same as those used in Example 1, except that heat treatment was at a set temperature of 650°C. CDS-1 was obtained as the product in this example (Figure 12).

### Example 9

### CDS-1 zeolite manufacture 1: heat treatment in vacuum

The conditions for synthesizing CDS-1 were the same as those used in Example 1, except that heat treatment was at a set temperature of 725°C. CDS-1 was obtained as the product in this example (Figure 12).

### Example 10

### CDS-1 zeolite manufacture 1: heat treatment in vacuum

The conditions for synthesizing CDS-1 were the same as those used in Example 1, except that heat treatment was at a set temperature of 800°C. CDS-1 was obtained as the product in this example (Figure 12).

### Example 11

### CDS-1 zeolite manufacture 1: heat treatment in vacuum

The conditions for synthesizing CDS-1 were the same as those used in Example 1, except that heat treatment was at a set temperature of 425°C. In this example a peak characteristic of CDS-1 was observed in the powder XRD pattern, but as shown in Figure 12, other diffraction patterns were observed to a certain extent, and these are thought to represent intermediates in the structural transformation.

### Example 12

### CDS-1 zeolite manufacture 1: heat treatment in vacuum

The conditions were the same as in Example 1 except that heat treatment was at preset temperatures of 200°C, 300°C and 350°C. Since in this example the powder XRD pattern was characteristic of the layered compound PLS-1, CDS-1 was not synthesized. This shows that as shown in Figure 12, the structural transformation of PLS-1 into CDS-1 occurs beginning at a heat treatment temperature of about 400°C.

### Example 13

### CDS-1 zeolite manufacture 2: Combustion-supporting gas heat treatment

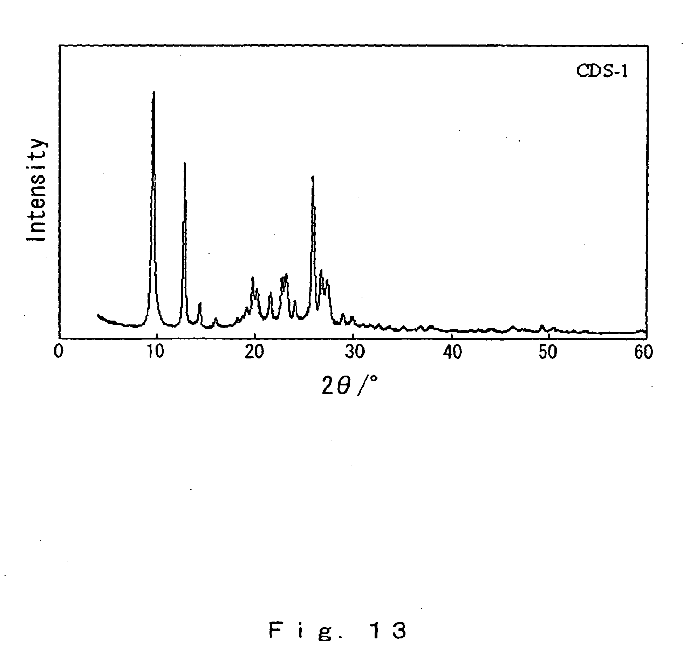

Figure 13 shows an XRD chart of the product when the temperature was raised to 400°C at a rate of 1°C a minute, and maintained at that temperature for 5 hours. This product has clearly changed into a crystalline compound different from the layered crystalline compound PLS-1 shown in Figure 12.

### Example 14

### CDS-1 zeolite manufacture 2: Combustion-supporting gas heat treatment

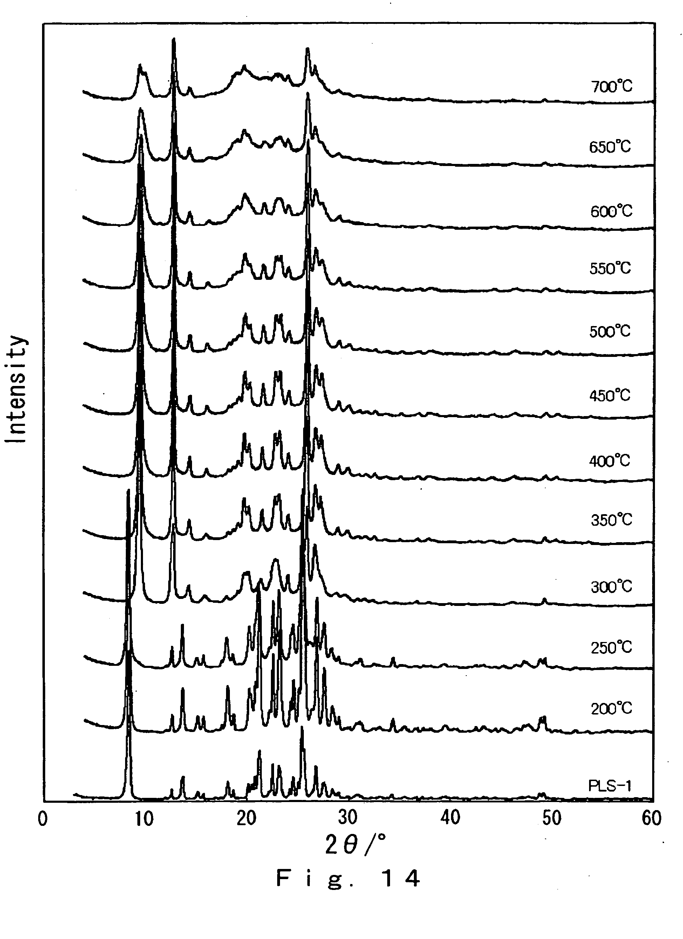

PLS-1 was subjected to dehydration polycondensation under atmospheric pressure in a flow of dry air. As shown in Figure 14, the dehydration temperature was between 200°C and 700°C, and the baking temperature was varied in 50°C increments. To obtain CDS-1 zeolite by this method, baking is preferably performed at between about 300°C and less than 600°C. Between 350°C and 500°C is more preferable.

It was confirmed that dehydration polycondensation of the layered silicate does not progress below 300°C. At temperatures of 600°C and higher, a structure attributed to CDS-1 is observed, but much amorphous material is also present and good CDS-1 is not obtained.

### Example 15

### CDS-1 zeolite manufacture 2: Combustion-supporting gas heat treatment

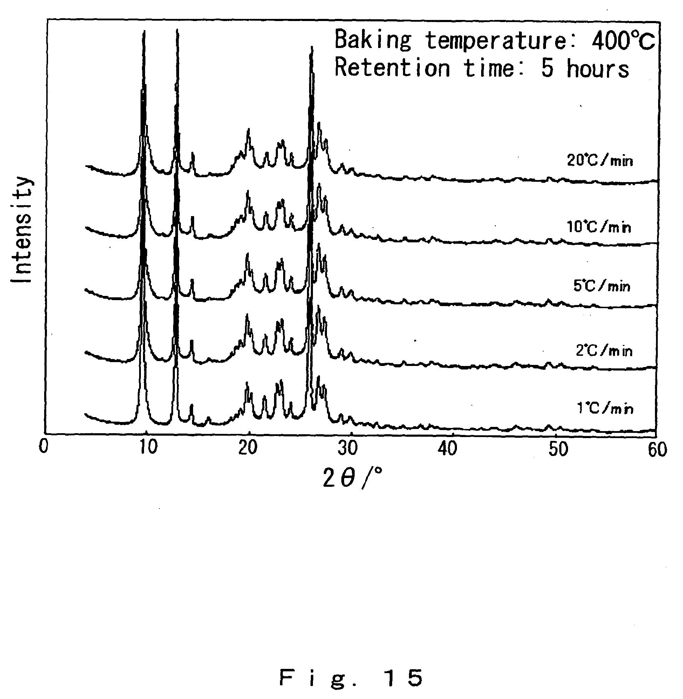

The rate of temperature rise is not particularly specified, but as shown in Figure 15 the temperature was raised to 400°C at rates of between 1 and 20°C per minute, and maintained at that temperature for 5 hours. The temperature should preferably be raised at 5°C/minute or less to prevent destruction of the crystal structure accompanying rapid dissociation and baking of the organic template in the layered crystalline silicate PLS-1.

No obvious differences in structure due to differences in rate of temperature increase were observed in powder x-ray crystal structure analysis of CDS-1 zeolites obtained at a heating temperature of 400°C.

At a heating temperature of 400°C, the XRD pattern was the same as the pattern of the CDS-1 shown in Example 13 even when the rate of temperature increase was 1°C/minute or less, confirming that CDS-1 was produced.

Moreover, at a heating temperature of 400°C, the XRD pattern was the same as that of the CDS-1 shown in Example 13 even when the rate of temperature increase was 20°C/minute or more, confirming that CDS-1 was produced.

### Example 16

### CDS-1 zeolite manufacture 2: Combustion-supporting gas heat treatment

The baking retention time is not particularly specified, and Figure 15 shows the XRD chart for different retention times when the temperature was raised to 400°C at 1°C/minute. Judging by this chart, there were no obvious differences in the structure of the resulting novel zeolite CDS-1 due to the baking retention time. However, some brownish powder derived from imperfectly combusted organic template is seen in the CDS-1 when the baking retention time is short, while with a longer retention time the CDS-1 is a nearly white powder.

It was confirmed that CDS-1 was obtained even with a retention time of 10 hours or more since the XRD pattern was the same as that of the CDS-1 shown in Example 13.

### Example 17

### CDS-1 zeolite manufacture 2: Combustion-supporting gas heat treatment

It was confirmed that CDS-1 was obtained even when baking was performed in a flow of pure oxygen, since the XRD pattern was the same as that of the CDS-1 shown in Example 13.

It was also confirmed that CDS-1 was obtained even when baking was performed in air without reduced pressure, since the XRD pattern was the same as that of the CDS-1 shown in Example 13.

### Example 18

### CDS-1 zeolite manufacture 2: Combustion-supporting gas heat treatment

To study the effects of increased scale, the temperature was raised to 400°C at a rate of 1°C/minute in a muffle furnace and maintained for 10 hours using 10.0 g of PLS-1. In this case, the flow rate of the combustion-supporting gas (dry air) was 13 L/minute. The resulting product was a light yellowish-brown powder sample, with a yield of 7.21 g. Production of CDS-1 was confirmed since the measured XRD pattern was the same as that of the CDS-1 shown in Example 13.

### Example 19

### CDS-1 zeolite manufacture 2: Combustion-supporting gas heat treatment

Using 9.51 g of PLS-1, the temperature was raised to 450°C at a rate of 1°C/minute in a muffle furnace, and maintained for 10 hours. The flow rate of the combustion-supporting gas (dry air) was 13 L/minute. A slightly yellowish powder sample was produced with a yield of 6.70 g. Production of CDS-1 was confirmed since the measured XRD pattern was the same as that of the CDS-1 shown in Example 13.

### Example 20

### CDS-1 zeolite manufacture 2: Combustion-supporting gas heat treatment

Using 5.27 g of PLS-1, the temperature was raised to 500°C at a rate of 1°C/minute in a muffle furnace, and maintained for 10 hours. The flow rate of the combustion-supporting gas (dry air) was 7 L/minute. A white powder was produced with a yield of 4.01 g. Production of CDS-1 was confirmed since the measured XRD pattern was the same as that of the CDS-1 shown in Example 13.

### Example 21

### Manufacture of metal-containing crystalline layered compound PLS-1 and CDS-1 zeolite

In the PLS-1 synthesis of Example 1, 0.030 g of Al(NO₃) 9H₂O (Wako Pure Chemical) was added to the raw materials for an Al molar ratio of 1% or less, with all other conditions the same. A product exhibiting the same powder XRD pattern as PLS-1 was obtained in this example 1. Furthermore, 0.025% (wt/wt%) Al was detected in ICP analysis of this product. This Al-containing PLS-1 was subjected to dehydration polycondensation by vacuum heating at 500°C, 5 × 10⁻⁶ torr as in the example. Based on powder XRD and NMR measurement, a product having the same structure as CDS-1 was produced in this example.

### Example 22

### Manufacture of metal-containing crystalline layered compound PLS-1 and CDS-1 zeolite

In the PLS-1 synthesis of Example 1, 0.030 g of Ga(NO₃) 8H₂O (Soekawa Physics and Chemistry) was added to the raw materials to obtain a Ga molar ratio of 1% or less, with all other conditions the same. A product exhibiting the same powder XRD pattern as PLS-1 was obtained in this example. 0.36% (wt/wt%) of Ga was detected in ICP analysis of this product. This Ga-containing PLS-1 was subjected to dehydration polycondensation by vacuum heating at 500°C, 5 × 10⁻⁶ torr as in the example 1. Based on powder XRD and NMR measurement, a product having the same structure as CDS-1 was produced in this example.

### Example 23

### Manufacture of metal-containing crystalline layered compound PLS-1 and CDS-1 zeolite

In the PLS-1 synthesis of Example 1, 0.069 g of Ce(NO₃) 6H₂O (Wako Pure Chemicals) was added to the raw materials to obtain a Ce molar ratio of 1% or less, with all other conditions the same. A product exhibiting the same powder XRD pattern as PLS-1 was obtained in this example. 0.51% (wt/wt%) of Ce was detected in ICP analysis of this product. This Ce-containing PLS-1 was subjected to dehydration polycondensation by vacuum heating at 500°C, 5 × 10⁻⁶ torr as in the example 1. Based on powder XRD and NMR measurement, a product having the same structure as CDS-1 appears to have been produced in this example.

### Example 24

### Manufacture of metal-containing crystalline layered compound PLS-1 and CDS-1 zeolite

In the PLS-1 synthesis of Example 1, 0.033 g of Fe(NO₃) 9H₂O (Wako Pure Chemicals) was added to the raw materials to obtain a Fe molar ratio of 1% or less, with all other conditions the same. A product exhibiting the same powder XRD pattern as PLS-1 was obtained in this example. 0.36% (wt/wt%) of Fe was detected in ICP analysis of this product. This Fe-containing PLS-1 was subjected to dehydration polycondensation by vacuum heating at 500°C, 5 × 10⁻⁶ torr as in the example 1. Based on powder XRD and NMR measurement, a product having the same structure as CDS-1 was produced in this example.

### Example 25

### Manufacture of metal-containing crystalline layered compound PLS-1 and CDS-1 zeolite

In the PLS-1 synthesis of Example 1, 0.008 g of LiNo₃ (Merck Japan) was added to the raw materials to obtain a Li molar ratio of 1% or less, with all other conditions the same. A product exhibiting the same powder XRD pattern as PLS-1 was obtained in this example. 0.032% (wt/wt%) of Li was detected in ICP analysis of this product. This Li-containing PLS-1 was subjected to dehydration polycondensation by vacuum heating at 500°C, 5 × 10⁻⁶ torr as in the example 1. Based on powder XRD and NMR measurement, a product having the same structure as CDS-1 was produced in this example.

### Example 26

### Manufacture of metal-containing crystalline layered compound PLS-1 and CDS-1 zeolite

In the PLS-1 synthesis of Example 1, 0.014 g of RbCl (Merck Japan) was added to the raw materials to obtain an Rb molar ratio of 1% or less, with all other conditions the same. A product exhibiting the same powder XRD pattern as PLS-1 was obtained in this example. 0.18% (wt/wt) of Rb was detected in ICP analysis of this product. This Rb-containing PLS-1 was subjected to dehydration polycondensation by vacuum heating at 500°C, 5 × 10⁻⁶ torr as in the example 1. Based on powder XRD and NMR measurement, a product having the same structure as CDS-1 appears to have been produced in this example.

Examples of the first aspect of the present invention are given below.

### Example 27

### (1) PLS seed crystal synthesis method

2.00 g of SiO₂ (Product name: Cab-O-Sil M5, CABOT Co.) was taken and 25.4 g of 26% TMAOH (tetramethylammonium hydroxide: Tokyo Chemical Industry, special grade), 10.0 g of 0.5 standard KOH, 68.6 g of H₂O and 100.0 g of 1,4-dioxane (Tokyo Chemical Industry, special grade) were added thereto and agitated well for 1 hour. The mixture was then transferred to an autoclave (content volume 300 ml, NAC Autoclave) having an inner Teflon™ tube, and heat treated for 10 days at 150°C. After being removed from the autoclave, this was washed thoroughly with ethanol and water, and dried for 12 hours at 70°C to obtain a powder product. The yield was about 100% relative to the SiO. This product was also confirmed by ²⁹Si-MAS NMR, SEM and XRD measurement to be the layered compound PLS. The lattice spacing d (Å) shown in Table 17, which is characteristic of PLS, was obtained from the powder XRD diffraction pattern of this product.

**Table 17**

| d(Å) |
|---|
| 10.46±0.1 |
| 8.38±0.1 |
| 7.34±0.1 |
| 7.00±0.1 |
| 6.51±0.1 |
| 6.45±0.1 |
| 5.86±0.5 |
| 5.82±0.5 |
| 5.66±0.5 |
| 5.23±0.5 |
| 5.07±0.5 |
| 4.90±0.5 |
| 4.75±0.5 |
| 4.57±0.5 |
| 4.40±0.5 |
| 4.35±0.5 |
| 4.26±0.5 |
| 4.19±0.5 |
| 4.00±0.5 |

### (2) PLS membrane synthesis method

This PLS powder was applied by rubbing to the surface of a mullite tube (product name: PM, 6 mm φ, 80 mm length, mean pore size 1.8 µm, Nikkato). The top and bottom of this mullite tube were sealed with Teflon™ tape, and it was fixed perpendicularly on a Teflon™ jig (pedestal 20 mm thick and 38 mm in diameter with 7 mm through hole). Next, the mullite tube fixed on the pedestal was placed at the bottom of an SUS autoclave (Makabe Giken) with a diameter of 40 mm and a content volume of 250 mL. Next, 0.5 g of PLS crystals were added to the PLS synthesis raw material solution used in the aforementioned PLS seed crystal synthesis, and thoroughly agitated. Only 200 mL of this raw material solution containing PLS crystals was transferred to an autoclave so that the mullite tube was completely immersed. Next, this autoclave was placed in a blast oven (Yamato Kagaku, DK300) which had been pre-heated to 160°C, and hydrothermally treated for 72 hours. The autoclave was then removed, and the mullite tube fixed on the pedestal was thoroughly water washed. An independent membrane was formed on the pedestal. The water-washed mullite tube was dried for 24 hours at 70°C to obtain a PLS membrane.

### (3) Conversion of PLS membrane into CDS-1 membrane

This PLS membrane was transferred to a baking oven (Yamato Kagaku F0300, equipped with forced exhaust system), the temperature was raised from room temperature to 450°C at a rate of 1°C/minute, and the membrane was baked for 10 hours at 450°C to convert it to a CDS-1 membrane. X-ray diffraction measurements taken to evaluate the degree of crystallization of the resulting CDS-1 membrane show diffraction peaks with the d values shown in Tables 18 and 19 and a diffraction peak from the mullite tube used as a support. X-ray diffraction measurements of the powder present in the autoclave showed only a diffraction peak with the d values shown in Table 18. The membrane thickness was observed by electron microscopy and found to be about 8 µm.

**Table 18**

| d(Å) | Relative strength (peak) |
|---|---|
| 9.17±0.05 | 100 |
| 6.86±0.05 | 35 |
| 6.11±0.05 | 5 |
| 5.50±0.05 | 4 |
| 4.84±0.05 | 1 |
| 4.70±0.05 | 1 |
| 4.58±0.05 | 3 |
| 4.44±0.05 | 7 |
| 4.35±0.05 | 7 |
| 4.09±0.05 | 6 |
| 3.88±0.05 | 8 |
| 3.81±0.05 | 9 |
| 3.68±0.05 | 3 |
| 3.43±0.05 | 16 |
| 3.41±0.05 | 18 |
| 3.31±0.05 | 8 |
| 3.24±0.05 | 9 |
| 3.07±0.05 | 1 |

**Table 19**

| d(Å) | Relative strength (peak) |
|---|---|
| 9.25±0.05 | 100 |
| 8.85±0.05 | 7 |
| 7.67±0.05 | 4 |
| 6.85±0.05 | 65 |
| 6.14±0.05 | 7 |
| 4.74±0.05 | 6 |
| 4.65±0.05 | 7 |
| 4.49±0.05 | 13 |
| 4.40±0.05 | 5 |
| 4.10±0.05 | 5 |
| 3.90±0.05 | 7 |
| 3.84±0.05 | 8 |
| 3.71±0.05 | 5 |
| 3.44±0.05 | 30 |
| 3.34±0.05 | 14 |
| 3.26±0.05 | 9 |
| 3.08±0.05 | 4 |
| 2.99±0.05 | 3 |
| 2.89±0.05 | 2 |
| 2.75±0.05 | 1 |
| 2.37±0.05 | 2 |
| 1.97±0.05 | 2 |
| 1.86±0.05 | 2 |

### Example 28

A CDS-1 membrane was synthesized under the same conditions and by the same operations as in Example 27 except that the conditions for hydrothermal synthesis of the PLS membrane were 150°C for 24 hours. In addition to peaks for the CDS-1 and the mullite tube support, the x-ray diffraction peaks for this membrane included a broad hollow peak in the range of 20-20° (CuKα). The membrane thickness was 1 µm or less, and electron microscopy showed that CDS-1 crystals were absent from some areas.

### Example 29

A CDS-1 membrane was synthesized under the same conditions and by the same operations as in Example 27 except that the conditions for hydrothermal synthesis of the PLS membrane were 150°C for 72 hours. In addition to peaks for the CDS-1 and the mullite tube support, the x-ray diffraction peaks for this membrane included a broad hollow peak in the range of 20-30° (CuKα). The membrane thickness was 1 µm or less, and electron microscopy showed that CDS-1 crystals were absent from some areas.

### Example 30

A CDS-1 membrane was synthesized under the same conditions and by the same operations as in Example 1 except that suctioning was adopted as the method of applying the PLS seed crystals to the mullite tube, and the PLS membrane hydrothermal synthesis conditions were 150°C for 24 hours. The method of suctioning the PLS seed crystals to the mullite tube surface was to add 0.5 g of PLS crystals to 100 g of ion exchange water, treat them for 10 minutes with an ultrasound cleaner (As One, US-4, output 500 W), immerse a mullite tube with one opening sealed with Teflon™ tape, and connect the other end to a vacuum pump to suction the PLS crystal solution, thereby applying the PLS crystals. Next, the mullite tube was dried for 24 hours at 70°C, and the PLS membrane was subjected to hydrothermal synthesis. The resulting membrane exhibited an x-ray diffraction peak only for the mullite tube support. Almost no CDS-1 crystals were observed by electron microcopy.

### Example 31

A CDS-1 membrane was synthesized under the same conditions and by the same operations as in Example 30 except that the conditions for hydrothermal synthesis of the PLS membrane were 150°C for 72 hours. The resulting membrane exhibited an x-ray diffraction peak for the mullite tube support and a broad hollow peak in the range of 20 to 30° (CuKα). Some areas lacked CDS-1 crystals as observed by electron microscopy.

### Example 32

A CDS-1 membrane was synthesized under the same conditions and by the same operations as in Example 30 except that the conditions for hydrothermal synthesis of the PLS membrane were 160°C for 72 hours. The resulting membrane exhibited an x-ray diffraction peak for the mullite tube support and a broad hollow peak in the range of 20 to 30° (CuKα). Some areas lacked CDS-1 crystals as observed by electron microscopy.

### Example 33

A CDS-1 membrane was synthesized under the same conditions and by the same operations as in Example 27 except that the porous support was an alumina tube (mean pore size 0.2 µm, NGK Insulators) and the conditions for hydrothermal synthesis of the PLS membrane were 150°C for 72 hours. The only x-ray diffraction peak observed was that of the alumina tube used as the support. Almost no CDS-1 crystals were observed by electron microscopy.

### Example 34

A CDS-1 membrane was synthesized under the same conditions and by the same operations as in Example 33 except that the conditions for hydrothermal synthesis of the PLS membrane were changed to 160°C for 240 hours. The only x-ray diffraction peak observed was that of the alumina tube used as the support. Almost no CDS-1 crystals were observed by electron microscopy.

### Example 35

A CDS-1 membrane was synthesized under the same conditions and by the same operations as in Example 33 except that suction was adopted instead of the method of implantation on the alumina tube. The only x-ray diffraction peak observed was that of the alumina tube used as the support. Almost no CDS-1 crystals were observed by electron microscopy.

### (Comparative Example 1)

A CDS-1 membrane was synthesized under the same conditions and by the same operations as in Example 27 except that the conditions for hydrothermal synthesis of the PLS membrane were changed to 130°C for 72 hours. The only x-ray diffraction peak observed was that of the mullite tube used as the support. Almost no CDS-1 crystals were observed by electron microscopy.

### (Comparative Example 2)

A CDS-1 membrane was synthesized under the same conditions and by the same operations as in Example 27 except that the conditions for hydrothermal synthesis of the PLS membrane were changed to 180°C for 72 hours. The only x-ray diffraction peak observed was that of the mullite tube used as the support. Almost no CDS-1 crystals were observed by electron microscopy.

### (Comparative Example 3)

A CDS-1 membrane was synthesized under the same conditions and by the same operations as in Example 27 except that suction was adopted instead of the method of implantation on the mullite tube, and the conditions for hydrothermal synthesis of the PLS membrane were changed to 130°C for 72 hours. The only x-ray diffraction peak observed was that of the alumina tube used as the support. Almost no CDS-1 crystals were observed by electron microscopy.

### (Comparative Example 4)

A CDS-1 membrane was synthesized under the same conditions and by the same operations as in Example 27 except that suction was adopted instead of the method of implantation on the mullite tube, and the conditions for hydrothermal synthesis of the PLS membrane were changed to 180°C for 72 hours. The only x-ray diffraction peak observed was that of the alumina tube used as the support. Almost no CDS-1 crystals were observed by electron microscopy.

The PLS membrane synthesis conditions for Examples 27 to 35 and Comparative Examples 1 to 4 are summarized in Table 20. Electron microscope images of the PLS membrane synthesized in Example 27 and the baked CDS-1 membrane are shown in Figure 2.

**[Table 20]**

| | Support | Implantation method | Hydrothermal synthesis conditions (temp/time) | Membrane thickness (µm) |
|---|---|---|---|---|
| Example 1 | PM | Rubbing | 160/72 | 8 |
| Example 2 | PM | Rubbing | 150/24 | ∼1 |
| Example 3 | PM | Rubbing | 150/72 | ∼1 |
| Example 4 | PM | Suction | 150/24 | ∼1 |
| Example 5 | PM | Suction | 150/72 | ∼1 |
| Example 6 | PM | Rubbing | 160/72 | ∼1 |
| Example 7 | NGK | Rubbing | 150/72 | ∼1 |
| Example 8 | NGK | Rubbing | 160/240 | ∼1 |
| Example 9 | NGK | Suction | 160/240 | ∼1 |
| Comparative Example 1 | PM | Rubbing | 130/72 | Not produced |
| Comparative Example 2 | PM | Rubbing | 180/72 | Not produced |
| Comparative Example 3 | PM | Suction | 130/72 | Not produced |
| Comparative Example 4 | PM | Suction | 180/72 | Not produced |

| | | | | |
|---|---|---|---|---|
| PM: Nikkato mullite tube NGK: NGK Insulators alumina tube Rubbing: Rubbing method Suction: Suction method (Gas permeation test) | | | | |

A permeation test using nitrogen gas was performed to evaluate the PLS membranes prepared in Examples 27 to 35. Both ends of the resulting PLS membrane were connected to a 1/4 inch SUS tube using Varian Torr Seal, and fixed to an SUS measurement tube with an inner diameter of 15 mm. The amount of nitrogen gas permeating from the outside to the inside of the PLS membrane was measured at room temperature with the supply side pressure at 0.2 MPs and the permeation side open to the air.

### (Examples 36 to 44)

Nitrogen gas permeation results for the PLS membranes obtained in Examples 39 to 35 are summarized in Table 21. It can be seen that a dense membrane which is not permeated by nitrogen gas can be produced by applying PLS seed crystals by rubbing and hydrothermally treating them for 72 hours at 160°C. It also appears that when implantation was by suction and by the alumina tube having a mean pore size of 0.2 µm, not enough seed crystals were applied for purposes of secondary growth, and there was insufficient adhesion with the support (PLS seed crystals are thin crystals of about 2 µm or less).

**[Table 21]**

| | Resulting PLS membrane | Nitrogen gas permeation (mol/m² · sec · Pa) |
|---|---|---|
| Example 10 | Example 1 | Below detection limit |
| Example 11 | Example 2 | 4.47 x 10⁻⁶ |
| Example 12 | Example 3 | 1.34 x 10⁻⁶ |
| Example 13 | Example 4 | 1.28 x 10⁻⁵ |
| Example 14 | Example 5 | 1.54 x 10⁻⁵ |
| Example 15 | Example 6 | 1.51 x 10⁻⁵ |
| Example 16 | Example 7 | 1.60 x 10⁻⁵ |
| Example 17 | Example 8 | 1.60 x 10⁻⁵ |
| Example 18 | Example 9 | 1.60 x 10⁻⁵ |

### (Example 45)

### (Ethanol/water separation by osmoticvaporation method)

Ethanol and water were separated by osmoticvaporation method using the CDS-1 membrane obtained in Example 27. One end of the CDS-1 membrane was sealed with a Torr seal (Varian), while the other was connected to a 1/4 inch SUS tube with a Torr seal. The SUS tube was then connected to a vacuum pump. The effective surface area of the CDS-1 membrane in this case was 7.15 cm². The supply liquid was a 2 vol% aqueous ethanol solution which had been heated to 40°C. The output side of the membrane was provided with a liquid nitrogen trap for collecting the permeated liquid. The ethanol/water ratio of the supply liquid and permeated liquid was analyzed by gas chromatography using a Polarpack Q column. As a result, the separation factor of water to ethanol was 23, and the permeation flow rate was 0.23 kg/m³ · h.

### (Example 46)

The same operations and tests as in Example 45 were repeated continuously for 24 hours, and the permeated liquid was collected. As a result, the separation factor of water to ethanol was 32, and the permeation flow rate was 0.27 kg/m³ · h. The water-selective permeation capability (hydrophilicity) was somewhat greater than in Example 45. It is thought that this membrane is a water-selective permeation membrane (hydrophilic membrane) because of the presence of residual TMA (tetramethylammonium) and Si-OH groups remaining in the membrane after condensation from PLS. The CDS-1 membrane of Example 1 was baked for 10 hours at a temperature of 450°C, but it is thought that the amounts of residual TMA and Si-OH groups can be controlled by changing the baking conditions, temperature and time, allowing the synthesis of a wide range of membranes with different properties, from hydrophilic membranes to hydrophobic membranes similar to silicalite.

### (Example 47)

Ethanol/water were separated by osmoticvaporation method using a CDS-1 membrane as in Example 45. The CDS-1 membrane was baked at 600°C for 10 hours. That is, all TMA and Si-OH groups were removed. The effective surface area of the CDS-1 membrane in this case was 6.59 cm². The supply liquid was a 5% vol. ethanol/water solution which had been heated to 40°C. The output side of the membrane was provided with a liquid nitrogen trap for collecting the permeated liquid. The ratio of ethanol to water in the supply liquid and permeated liquid was analyzed by column chromatography using a Porapak Q column. As a result, the separation factor of water to ethanol was found to be 0.0188, with a permeation flow rate of 0.53 kg/m³ · h. That is, a hydrophobic membrane rather than a hydrophilic membrane can be synthesized by changing the baking conditions.

### (Example 48)

PLS obtained by the aforementioned method for synthesizing PLS seed crystals was placed in a glass tube with an inner diameter of 25 mm which was then attached to a vacuum line, and subjected to a 3-step heat treatment in a vacuum of 5 × 10⁻³ in which the temperature was raised from room temperature to 500°C over the course of 4 hours, maintained for 4 hours and reduced to room temperature over the course of 1 hour, to obtain CDS-1 zeolite as a gray powder. This CDS-1 powder was rubbed as the seed crystals on a PM mullite tube. This was then hydrothermally treated as in Example 27. However, the hydrothermal treatment solution consisted of 2 g of 1 standard KOH aqueous solution and 2 g of TMAOH (tetramethylammonium hydroxide: Tokyo Chemical Industry, special grade) at 26% concentration added to 150 mL of ion exchange water. Hydrothermal treatment was performed for 24 hours at 150°C. After hydrothermal treatment, the mullite tube was thoroughly water washed and dried for 24 hours at 70°C, the temperature was raised from room temperature to 450°C at a rate of 1°C/minute, and the tube was baked for 10 hours at 450°C. The thickness of this membrane as observed under an electron microscope was about 3 µm.

### (Example 49)

The hydrothermal treatment as same as in Example 48 was performed except that the condition of the hydrothermal treatment was for 48 hours at 150°C. The membrane thickness was about 5 µm.

Examples of the second aspect of the present invention are given next.

### Manufacturing Example 1

### (1) Layered compound PLS-1

10.0 g of SiO₂ (Produce name: Cab-O-Sil M5, CABOT Co.) was taken and added to 22.0 g of 15% TMAOH (tetramethylammonium hydroxide), 5.0 g of 0.5 standard KOH, 25.0 g of H₂O and 50.0 g of 1,4-dioxane, and agitated thoroughly for 1 hour, and then transferred to an SUS316 autoclave with a content volume of 300 ml having a Teflon™ inner cylinder, and heat treated for 10 days at 150°C. After being removed from the autoclave, this was washed with acetone and water, and dried for 12 hours at 70°C to obtain a product in powder form.

²⁹Si-MAS NMR, SEM and XRD measurement were used to confirm that this product was the layered compound PLS-1. The lattice spacing d (Å) shown in Table 22, which is characteristic of PLS-1, was obtained from the powder x-ray diffraction pattern of this product.

**Table 22**

| d(Å) |
|---|
| 10.46±0.1 |
| 8.38±0.1 |
| 7.34±0.1 |
| 7.00±0.1 |
| 6.51±0.1 |
| 6.45±0.1 |
| 5.86±0.05 |
| 5.82±0.05 |
| 5.66±0.05 |
| 5.23±0.05 |
| 5.07±0.05 |
| 4.90±0.05 |
| 4.75±0.05 |
| 4.57±0.05 |
| 4.40±0.05 |
| 4.35±0.05 |
| 4.26±0.05 |
| 4.19±0.05 |
| 4.00±0.05 |

### (2) Manufacture of CDS-1 zeolite

This PLS-1 was placed in a glass tube with an inner diameter of 25 mm which was then connected to a vacuum line, and subjected to a 3-step heat treatment in which the temperature was raised from room temperature to 500°C over the course of 4 hours, maintained in a vacuum of 5 x 10⁻⁶ for 4 hours and then cooled to room temperature over the course of 1 hour to obtain a CDS-1 zeolite product as a gray powder. The crystals were thin and scale-shaped, 1 to 2 µm on a side with a thickness of 0.5 µm, and it appeared that CDS-1 had been produced by a structural change geometrically analogous to that of the layered structure PLS-1.

### Manufacturing Example 2

### Manufacture of CDS-1 zeolite

CDS-1 was synthesized as in Manufacturing Example 1 except that heat treatment was set at temperature of 575°C. CDS-1 was obtained as the product in this manufacturing example.

### Manufacturing Example 3

### Manufacture of CDS-1 zeolite

CDS-1 was synthesized as in Manufacturing Example 1 except that heat treatment was set at temperature of 650°C. CDS-1 was obtained as the product in this manufacturing example.

### Manufacturing Example 4

### Manufacture of CDS-1 zeolite

CDS-1 was synthesized as in Manufacturing Example 1 except that heat treatment was set at temperature of 725°C. CDS-1 was obtained as the product in this manufacturing example.

### Manufacturing Example 5

### Manufacture of CDS-1 zeolite

CDS-1 was synthesized as in Manufacturing Example 1 except that heat treatment was set at temperature of 800°C. CDS-1 was obtained as the product in this manufacturing example.

### Manufacturing Example 6

### Manufacture of CDS-1 zeolite

CDS-1 was synthesized under the same conditions as in Manufacturing Example 1 except that heat treatment was set at temperature of 425°C. In this manufacturing example, peaks characteristic of CDS-1 were observed in the powder XRD pattern, but some other diffraction peaks were also observed, so the product appeared to be an intermediate in the structural transformation.

### Manufacturing Example 7

### Manufacture of CDS-1 zeolite

CDS-1 was synthesized under the same conditions as in Manufacturing Example 1 except that heat treatment was set at temperatures of 200°C, 300°C and 350°C. In this manufacturing example, a powder XRD pattern characteristic of the original layered compound PLS-1 was observed, and no CDS-1 was synthesized. This shows that the structural transformation from PLS-1 to CDS-1 occurs when a heat treatment temperature is over about 400°C.

The effectiveness of CDS-1 for ε-caprolactam synthesis by Beckmann rearrangement is explained in detail below using examples.

### Catalysis test

A vapor phase Beckmann rearrangement reaction of cyclohexanone oxime into ε-caprolactam was accomplished in a stationary reaction vessel with an inner diameter of 8 mm. When the catalyst was a powder it was used mixed with quartz wool. Granular catalysts were first pressure molded, then pulverized and prepared as 10 to 20 mesh. 0.5 g of catalyst was used. Most catalyst layers were about 30 mm in length. A 5% cyclohexanone oxime solution was vaporized, and supplied to the reaction vessel together with a carrier gas (nitrogen). The main reaction conditions were normal pressure and 300 to 400°C, with the space velocity WHSV of the cyclohexanone oxime at between 0.025 h-1 and 25 h-1. The reaction product was collected by cooling with dry ice methanol. The resulting reaction product was analyzed by gas chromatography (J & W Scientific DB-1701, Length 30 m, φ0.25 mm) using methyl undecanate as the internal standard. The product was verified by GCMS (Varian Inova 500). The solid acid point was measured by the ammonia TPD (Japan Bell TPD-1AT (TCD Dec.)) method.

The cyclohexanone oxime conversion rate and ε-caprolactam selectivity were derived from the following formulae: conversion rate (%) = [(X - Y)/X] × 100, selectivity (%) = [Z/(X - Y)] × 100, with X, Y and Z defined as follows:
X = Number of moles of raw material cyclohexanone oxime
Y = Number of moles of unreacted cyclohexanone oxime
Z = Number of moles of ε-caprolactam product

### (Example 50)

Using 0.5 g of CDS-1 (H exchanged type) as the catalyst at a reaction temperature of 355°C, a raw material 5% cyclohexanone oxime methanol solution was supplied at 0.025 ml/min and reacted with 10 ml/min of nitrogen supplied as the sweep gas. The cyclohexanone oxime conversion rate was 75% in this case, and the ε-caprolactam selectivity was about 75% or more.

### (Example 51)

Using 0.5 g of CDS-1 (H exchanged type) as the catalyst at a reaction temperature of 364°C, a raw material 5% cyclohexanone oxime methanol solution was supplied at 0.025 ml/min and reacted with 10 ml/min of nitrogen supplied as the sweep gas. The cyclohexanone oxime conversion rate was 99% in this case, and the ε-caprolactam selectivity was about 76% or more.

### (Example 52)

Using 0.5 g of a granular shaped product (10 mesh) of CDS-1 (H exchanged type) at a reaction temperature of 364°C, a raw material 5% cyclohexanone oxime methanol solution was supplied at 0.025 ml/min and reacted with 10 ml/min of nitrogen supplied as the sweep gas. The cyclohexanone oxime conversion rate was 100% in this case, and the ε-caprolactam selectivity was about 54.8% or more.

### (Example 53)

Using 0.5 g of a granular shaped product (10 mesh) of CDS-1 (cationic type) at a reaction temperature of 364°C, a raw material 5% cyclohexanone oxime methanol solution was supplied at 0.025 ml/min and reacted with 10 ml/min of nitrogen supplied as the sweep gas. The cyclohexanone oxime conversion rate was 45.6% in this case, and the ε-caprolactam selectivity was about 49% or more.

### (Example 54)

Using 0.5 g of a granular shaped product of CDS-1 (cationic type) at a reaction temperature of 355°C, a raw material 5% cyclohexanone oxime methanol solution was supplied at 0.025 ml/min and reacted with 10 ml/min of nitrogen supplied as the sweep gas. The cyclohexanone oxime conversion rate was 40% in this case, and the ε-caprolactam selectivity was about 80% or more.

### (Example 55)

A reaction was performed as in Example 1 except that the injection rate of the raw material 5% cyclohexanone oxime methanol solution was tripled to 0.075 ml/min, using 0.5 g of CDS-1 (H exchanged type) as the catalyst at a reaction temperature of 355°C with nitrogen supplied at 10 ml/min as the sweep gas. In this case the cyclohexanone oxime conversion rate was 87.7%, and the ε-caprolactam selectivity was about 63.8% or more.

### (Example 56)

Except that the reaction temperature was raised to 370°C, the reaction was performed as in Example 6, that is, using 0.5 g of CDS-1 (H exchanged type) as the catalyst, with the 5% cyclohexanone oxime methanol solution injection rate tripled to 0.075 ml/min, and with nitrogen supplied at 10 ml/min as the sweep gas. In this case the cyclohexanone oxime conversion rate was 98%, and the ε-caprolactam selectivity was about 37.8% or more.

### (Example 57)

Nitrogen was supplied at 30 ml/min as the sweep gas, and otherwise the reaction was performed using 0.5 g of CDS-1 (H exchanged type) as the catalyst at a reaction temperature of 355°C, with the raw material 5% cyclohexanone oxime methanol solution supplied at 0.025 ml/min. In this case the cyclohexanone oxime conversion rate was 45%, and the ε-caprolactam selectivity was about 40% or more.

### Comparative Example

Using 0.5 g of commercial MFI (silica:alumina ratio = 700 or more) as the catalyst at a reaction temperature of 350°C, a reaction was performed with the raw material 5% cyclohexanone oxime solution supplied at 0.025 ml/min and nitrogen supplied at 10 ml/min as the sweep gas. In this case the cyclohexanone oxime conversion rate was 40%, and the ε-caprolactam selectivity was about 50% or more.

### INDUSTRIAL APPLICABILITY

As discussed above, the present invention relates to a novel zeolite, and provides a zeolite having a novel crystal structure that is inexpensive and has a high silica content and micropores. This zeolite can be applied to solids for metal carrier, separation and adsorption agents, shape-selective solid catalysts, ion exchange agents, chromatography filler materials, chemical reaction sites and the like. The CDS-1 zeolite having a novel crystal structure can be formed easily, cheaply and efficiently with a simple operation. The method of the present invention is a method of obtaining a higher-level structure by dehydration polycondensation of the skeletal structure of a precursor, and thus provides a new tool for the structural design of novel zeolites at the atomic level, something which was extremely difficult in the past. The resulting zeolites may have different physical and chemical properties, and the method of the present invention provides a concept for new preparation methods in zeolite synthesis. Conversion to zeolite is accomplished at relatively low temperatures, opening the possibility of introducing metal oxides which are broken down at high temperatures, and thus allowing the manufacture of novel skeletally substituted zeolites. The present invention allows zeolites with novel structures and functions to be provided cheaply by easy methods, and allows the creation of new technologies and industries in the field of zeolite applications.

The present invention also allows conversion to a CDS-1 membrane in a simple process, such as for example hydrothermal synthesis of PLS seed crystals followed by condensation of Si-OH groups in the resulting PLS membrane. Not only does the present invention allow the easy and rapid manufacture of zeolite membranes which can be applied to industrial liquid and gas separation processes and the like, but the resulting zeolite membranes can be applied favorably as membrane reactors with both separation and catalytic functions in the petrochemical industry for example.

Moreover, the present invention can provide a novel Beckmann rearrangement reaction by which ε-caprolactam can be manufactured highly efficiently from cyclohexanone oxime using a novel silica zeolite catalyst. Because the CDS-1 used in the present invention can use as the structure directing agent low-molecular-weight, inexpensive tetramethylammonium hydroxylate, which can also be recovered and reused, using this CDS-1 allows the construction of a synthesis process which is cheaper than conventional methods. Unlike ordinary alumina-containing zeolites this CDS-1 has only a silica source as its raw material, and lacks the acid properties resulting from binding between alumina and silica, so that a reaction method different from conventional reactions using solid acid properties is provided by the present invention. Because fuming sulfuric acid is mainly used as the catalyst in conventional reactions, a large quantity of ammonium sulfate is produced as a bi-product after neutralization with ammonia, but the present invention provides a clean reaction process which does not generate such unnecessary bi-products. The present invention allows the establishment of an ε-caprolactam production system using an economical, environmentally-friendly synthesis process.

## Claims

1. A zeolite **characterized by** having the chemical composition (structural composition of crystals) represented by [(Si₃₆₋ₓT_{y}O₇₂)M_{z}] (wherein M is a cation of an alkali metal selected from Li, Na, K or Rb, T represents A1, Ga, or Fe as skeleton substituting elements, x satisfies 0 ≤ x ≤ 3.0, y satisfies 0 ≤ y ≤ 1.0, and z satisfies 0 ≤ z ≤ 3.0), and having a micropore structure made up of covalent bonds between Si and O atoms.

2. The zeolite according to Claim 1, wherein the lattice spacing d (Å) in the powder x-ray diffraction pattern is as described in Tables 2 or 3 below.
**Table 2**
| d (Å) Relative | strength |
|---|---|
| 9.17 ± 0.05 | 100 |
| 6.86 ± 0.05 | 35 |
| 6.11 ± 0.05 | 5 |
| 5.50 ± 0.05 | 4 |
| 4.84 ± 0.05 | 1 |
| 4.70 ± 0.05 | 1 |
| 4.58 ± 0.05 | 3 |
| 4.44 ± 0.05 | 7 |
| 4.35±0.05 | 7 |
| 4.09±0.05 | 6 |
| 3.88±0.05 | 8 |
| 3.81±0.05 | 9 |
| 3.68±0.05 | 3 |
| 3.43±0.05 | 25 |
| 3.41±0.05 | 29 |
| 3.31±0.05 | 8 |
| 3.24±0.05 | 9 |
| 3.07±0.05 | 1 |
**Table 3**
| d(Å) | Relative strength |
|---|---|
| 9.25±0.05 | 100 |
| 8.85±0.05 | 7 |
| 7.67±0.05 | 4 |
| 6.85±0.05 | 65 |
| 6.14±0.05 | 7 |
| 4.74±0.05 | 6 |
| 4.65±0.05 | 7 |
| 4.49±0.05 | 13 |
| 4.40±0.05 | 5 |
| 4.10±0.05 | 5 |
| 3.90±0.05 | 7 |
| 3.84±0.05 | 8 |
| 3.71±0.05 | 5 |
| 3.44±0.05 | 30 |
| 3.34±0.05 | 14 |
| 3.26±0.05 | 9 |
| 3.08±0.05 | 4 |
| 2.99±0.05 | 3 |
| 2.89±0.05 | 2 |
| 2.75±0.05 | 1 |
| 2.37±0.05 | 2 |
| 1.97±0.05 | 2 |
| 1.86±0.05 | 2. |

3. The zeolite according to Claim 1, wherein the crystal structures can be described as orthorhombic with crystal lattice constants in the range of a = 18.35 ± 0.05 A, b = 13.77 ± 0.03, c = 7.37 ± 0.03 Å (space group Puma), orthorhombic with lattice constants in the range of a = 18.35 ± 0.05 Å, b = 13.77 ± 0.03, c = 7.37 ± 0.03 Å (space group Pnnm), orthorhombic with lattice constants in the range of a = 18.35 ± 0.05 Å, b = 13.77 ± 0.03, c = 14.74 ± 0.03 Å (space group Pbcm) or monoclinic with lattice constants in the range of a =18.35 ± 0.05A, b = 13.77 ± 0.03, c = 7.37 ± 0.03 Å, β= 90 ± 0.3°. (space group P21/m).

4. The zeolite according to Claim 1, wherein the local coordination of the O atoms surrounding the Si atoms in the skelton structure is tetracoordinate.

5. The zeolite according to Claim 1, wherein the skeletal structure formed by the binding of the Si and O atoms has a regular geometry.

6. The zeolite according to Claim 1, having pores with a mean size of 0.48 nm or more measured by a gas adsorption method.

7. A method for manufacturing a zeolite **characterized by** performing dehydration polycondensation of the crystalline layered compound or crystalline layered compound
containing skeletal substituted elements said crystalline layered compound having a chemical composition represented by [(Si₁₈₋ₓ O₃₈) M_{y} (TMA), (H₂O)_{w}] (wherein TMA is a tetraalkylammonium cation, M is a cation of an alkali metal selected from Na, K or Li, x satisfies 0 ≤ x ≤ 1.2, y satisfies 0.5 ≤ y ≤ 1.5, z satisfies 6 ≤ z ≤ 8, and w satisfies 0.02 ≤ w ≤ 1.5), having as the basic structure thereof a single-layer skeleton comprising one-dimensional micropores nanometers in size formed by a network of covalent bonds between Si and O atoms, the lattice spacing d in the powder x-ray diffraction pattern being at least as described in Table 1 below (wherein d is the lattice spacing, w = weak relative strength, m = moderate relative strength, s = strong relative strength and vs = very strong relative strength):
**Table 1**
| d(Å) | Relative strength |
|---|---|
| 10.47±0.2 | vs |
| 8.38±0.15 | w |
| 7.34±0.15 | m |
| 7.00±0.1 | m |
| 6.51±0.1 | m |
| 6.45±0.1 | s |
| 5.86±0.05 | m |
| 5.82±0.04 | m |
| 5.66±0.04 | w |
| 5.23±0.04 | m |
| 5.07±0.04 | w |
| 4.90±0.04 | s |
| 4.75±0.04 | m |
| 4.57±0.04 | w |
| 4.40±0.04 | m |
| 4.35±0.04 | s |
| 4.26±0.04 | s |
| 4.19±0.04 | vs |
| 4.00±0.04 | m |
| 3.94±0.035 | s |
| 3.85±0.035 | s |
| 3.83±0.035 | vs |
| 3.78±0.035 | w |
| 3.67±0.035 | m |
| 3.63±0.035 | s |
| 3.60±0.035 | w |
| 3.55±0.035 | m |
| 3.51±0.035 | m |
| 3.50±0.035 | vs |
| 3.48±0.035 | vs |
| 3.38±0.035 | m |
| 3.34±0.035 | w |
| 3.32±0.035 | s, |
to synthesize a zeolite with the chemical composition represented by [(Si₃₆₋ₓT_{y}O₇₂) M_{z}] (wherein M is a cation of an alkali metal selected from Li, Na, K or Rb, T represents Al, Ga, or Fe as skeleton substituting elements, x satisfies 0 ≤ x ≤ 3.0, y satisfies 0 ≤ y ≤ 1.0 and z satisfies 0 ≤ z ≤ 3.0).

8. The method for manufacturing a zeolite according to Claim 7, wherein manufacture is in a vacuum in the range of 1 x 10⁻³ to 1 x 10⁻⁸ torr as a condition for dehydration polycondensation.

9. The method for manufacturing a zeolite according to Claim 7, wherein the heating temperature for dehydration polycondensation is 400 to 800°C.

10. The method for manufacturing a zeolite according to Claim 7, wherein the zeolite is manufactured at atmospheric pressure as a condition for dehydration polycondensation.

11. The method for manufacturing a zeolite according to Claim 7, wherein the heating temperature for dehydration polycondensation is 300 to 800°C.

12. The method for manufacturing a zeolite according to Claim 7, wherein the rate of temperature rise is 0.5 to 50°C per minute.

13. The method for manufacturing a zeolite according to Claim 7, wherein dehydration polycondensation is performed with a flow of combustion-supporting gas a gas comprising oxygen molecules in a molecular state.

14. A zeolite membrane **characterized by** comprising a zeolite (CDS-1) formed as a membrane on a support, said zeolite having the chemical composition represented by [(Si₃₆₋ₓ O₇₂) M_{y}] (wherein M is a cation of an alkali metal selected from Na, K or Li, x satisfies 0 ≤ x ≤ 3.0, y satisfies 0 ≤ y ≤ 3.0) and a micropore structure made up of covalent bonds between Si and O atoms, with a silicate structure of repeating units of Si-O tetrahedral coordination and geometrical crystal structures (atomic arrangement) comprising silicon 5-member and 8-member rings.

15. The zeolite membrane according to Claim 14, wherein said crystal structures are (1) orthorhombic with crystal lattice constants in the range of a = 18.35 ± 0.05 A, b = 13.77 ± 0.03, c = 7.37 ± 0.03Å (space group Pnma),(2) orthorhombic with lattice constants in the range of a = 18.35 ± 0.05 Å, b = 13.77 ± 0.03, c = 7.37 ± 0.03 Å (space group Pnnm),(3) orthorhombic with lattice constants in the range of a = 18.35 ± 0.05 Å, b = 13.77 ± 0.03, c = 14.74 Å ± 0.03 Å (space group Pbmn) or (4) monoclinic with lattice constants in the range of a = 18.35 ± 0.05 Å, b = 13.77 ± 0.03, c = 7.37 ± 0.03 Å, = 90 ±0.3°. (F21/m).

16. The zeolite membrane according to Claim 14, wherein the lattice spacing d (Å) in the powder x-ray diffraction pattern is at least as described in Tables 4 and 5.
**Table 4**
| d(Å) | Relative strength (peak) |
|---|---|
| 9.17±0.05 | 100 |
| 6.86±0.05 | 35 |
| 6.11±0.05 | 5 |
| 5.50 0±.05 | 4 |
| 4.84±0.05 | 1 |
| 4.70±0.05 | 1 |
| 4.58±0.05 | 3 |
| 4.44±0.05 | 7 |
| 4.35±0.05 | 7 |
| 4.09±0.05 | 6 |
| 3.88±0.05 | 8 |
| 3.81±0.05 | 9 |
| 3.68±0.05 | 3 |
| 3.43±0.05 | 16 |
| 3.41±0.05 | 18 |
| 3.31 ± 0.05 | 8 |
| 3.24 ± 0.05 | 9 |
| 3.07 ± 0.05 | 1 |
**Table 5**
| d(Å) | Relative strength (peak) |
|---|---|
| 9.25 ± 0.05 | 100 |
| 8.85 ± 0.05 | 7 |
| 7.67 ± 0,05 | 4 |
| 6.85 ± 0.05 | 65 |
| 6.14 ± 0.05 | 7 |
| 4.74 ± 0.05 | 6 |
| 4.65 ± 0.05 | 7 |
| 4.49 ± 0.05 | 13 |
| 4.40 ± 0.05 | 5 |
| 4.10 ± 0.05 | 5 |
| 3.90 ± 0.05 | 7 |
| 3.84 ± 0.05 | 8 |
| 3.71 ± 0.05 | 5 |
| 3.44 ± 0.05 | 30 |
| 3.34 ± 0.05 | 14 |
| 3.26 ± 0.05 | 9 |
| 3.08 ± 0.05 | 4 |
| 2.99 ± 0.05 | 3 |
| 2.89 ± 0.05 | 2 |
| 2.73 ± 0.05 | 1 |
| 2.37 ± 0.05 | 2 |
| 1.97 ± 0.05 | 2 |
| 1.86 ± 0.05 | 2. |

17. The zeolite membrane according to Claim 14, wherein the support is a porous base of an inorganic porous body, metal or metal oxide.

18. A zeolite membrane manufacturing method **characterized by** using as seed crystals a crystalline layered silicate (hereunder abbreviated as PLS), the chemical composition of which is represented by [(Si₁₈₋ₓ O₃₈) M_{y}(TMA)_{z} (H₂O)_{w}] (wherein TMA is a tetraalkylammonium cation, M is a cation of an alkali metal x satisfies 0 ≤ x ≤ 1.2, y satisfies 0.5 ≤ y ≤ 1.5, z satisfies 6 ≤ z ≤ 8 and w satisfies 0.02 ≤ w ≤ 1.5), and having as the basic structure thereof a single-layer silicate skeleton comprising one-dimensional micropores nanometers in size formed by a network of covalent bonds between Si and O atoms, condensing the Si-OH groups in the PLS to converting the PLS to CDS-1 having a geometrical crystal structure (atomic arrangement) comprising silicon 5-member and 8-member rings, and thereby forming a zeolite membrane on a support.

19. The zeolite membrane manufacturing method according to Claim 18, wherein a PLS membrane is formed using PLS seed crystals.

20. The zeolite membrane manufacturing method according to Claim 18, wherein the support is a porous base of an inorganic porous body, metal or metal oxide.

21. The zeolite membrane manufacturing method according to Claim 19, wherein the PLS membrane is heated to 300°C to 800°C to condense the Si-OH groups in the PLS and convert to CDS-1.

22. The zeolite membrane manufacturing method according to Claim 21, wherein the PLS membrane is heated under reduced pressure.

23. The zeolite membrane manufacturing method according to Claim 19, wherein the PLS membrane is formed by hydrothermal synthesis at a temperature of 140 to 170°C.

24. The CDS-1 zeolite membrane manufacturing method according to Claim 18, wherein CDS-1 crystals synthesized from PLS are first applied to a support, and a membrane is then formed by secondary growth of the crystals.

25. A method for manufacturing ε-caprolactam from cyclohexanone oxime ε-caprolactam, **characterized in that** a zeolite (CDS-1) having the chemical composition represented by [(Si₃₆₋ₓT_{y} O₇₂) M_{z}] (wherein M is a cation of an alkali metal selected from Li, Na, K or Rb, T represents Al, Ga, Fe and Ce as skeleton substituting elements, x satisfies 0 ≤ x ≤ 3.0, y satisfies 0 ≤ y ≤ 1.0 and z satisfies ≤ z ≤ 3.0), and having a micropore structure made up of covalent bonds between Si and O atoms, and a geometric crystal structure (atomic arrangement) comprising silicon 5-member and 8-member rings is used as a catalyst.

26. The method for manufacturing ε-caprolactam according to Claim 25, wherein CDS-1 obtained by dehydration polycondensation at atmospheric pressure is used.

27. The method for manufacturing ε-caprolactam according to Claim 25, wherein CDS-1 obtained by dehydration polycondensation at a heating temperature of 300 to 800°C is used.

28. The method for manufacturing -caprolactam according to Claim 25, wherein CDS-1 I obtained by dehydration polycondensation with a rate of temperature rise of 0.1 to 10 °C/minuto is used.

29. The method for manufacturing ε-caprolactam according to Claim 25, wherein Cods- I obtained by treating the crystalline layered silicate compound which is the precursor with a group 6 transitional metal oxide in the CDS-1 synthesis process is used.

30. The method for manufacturing ε-caprolactam according to Claim 25, wherein the lattice spacing d (A) in the powder x-ray diffraction pattern of the CDS-1 exhibits at least the diffraction peaks given in Table 6 below.
**Table 6**
| d(Å) | Relative strength (peak) |
|---|---|
| 9,17 ± 0,05 | 140 |
| 6,86 ± 0,05 | 35 |
| 6,11 ± 0,05 | 5 |
| 5,50 ± 0,05 | 4 |
| 4,58 ± 0,05 | 3 |
| 4,44 ± 0,05 | 7 |
| 4,35 ± 0,05 | 7 |
| 4,09 ± 0,03 | 6 |
| 3,88 ± 0,05 | 8 |
| 3,81 ± 0,05 | 9 |
| 3,68 ± 0,05 | 3 |
| 3,43 ± 0,05 | 16 |
| 3,41 ± 0,05 | 18 |
| 3,31 ± 0,05 | 8 |
| 3,24 ± 0,05 | 9. |

31. The method for manufacturing ε-caprolaotam according to Claim 25, wherein the CDS-1 has micropores with a mean pore size of 0.483 nm or more based on physical adsorption and a volume of 0.6 cc/g or more.

32. The method for manufacturing ε-caprolactam according to Claim 25, wherein the CDS-1 used in the Beckmann rearrangement reaction is cation exchanged or hydrogen ion exchanged.

33. The method for manufacturing ε-caprolactam according to Claim 25, wherein the reaction temperature in the method for manufacturing ε-caprolactam from cyclohexanone oxime is 150 to 500°C.

34. The method for manufacturing ε-caprolactam according to Claim 25, wherein the WHSV of the cyclohexanone oxime is between 0.001 h-1 and 20.0 h-1.

## Patentansprüche

1. Zeolith, **dadurch gekennzeichnet, dass** er über eine durch [(Si₃₆₋ₓTyO₇₂)M_{z}] dargestellte chemische Zusammensetzung (strukturelle Zusammensetzung von Kristallen) verfügt (worin M ein Kation eines Alkalimetalls ist, das ausgewählt ist aus Li, Na, K oder Rb; T Al, Ga oder Fe als Skelett substituierende Elemente repräsentiert; x der Gleichung 0 ≥ x ≥ 3,0 genügt, y der Gleichung 0 ≥ y ≥ 1,0 genügt und z der Gleichung 0 ≥ z ≥ 3,0 genügt), wobei die Zusammensetzung eine Struktur von Mikroporen aufweist, die aus kovalenten Bindungen zwischen den Atomden Si und O aufgebaut ist.

2. Zeolith nach Anspruch 1, in welchem der Gitterabstand d (Å) im Röntgenbeugungsdiagramm nach dem Pulververfahren Werte hat, wie sie in den nachfolgenden Tabellen 2 oder 3 beschrieben werden.
**Tabelle 2**
| d (Å) | relative Intensität |
|---|---|
| 9,17 ± 0,05 | 100 |
| 6,86 ± 0,05 | 35 |
| 6,11 ± 0,05 | 5 |
| 5,50 ± 0,05 | 4 |
| 4,84 ± 0,05 | 1 |
| 4,70 ± 0,05 | 1 |
| 4,58 ± 0,05 | 3 |
| 4,44 ± 0,05 | 7 |
| 4,35 ± 0,05 | 7 |
| 4,09 ± 0,05 | 6 |
| 3,88 ± 0,05 | 8 |
| 3,81 ± 0,05 | 9 |
| 3,68 ± 0,05 | 3 |
| 3,43 ± 0,05 | 25 |
| 3,41 ± 0,05 | 29 |
| 3,31 ± 0,05 | 8 |
| 3,24 ± 0,05 | 9 |
| 3,07 ± 0,05 | 1 |
**Tabelle 3**
| d(Å) | relative Intensität |
|---|---|
| 9,25 ± 0,05 | 100 |
| 8,85 ± 0,05 | 7 |
| 7,67 ± 0,05 | 4 |
| 6,85 ± 0,05 | 65 |
| 6,14 ± 0,05 | 7 |
| 4,74 ± 0,05 | 6 |
| 4,65 ± 0,05 | 7 |
| 4,49 ± 0,05 | 13 |
| 4,40 ± 0,05 | 5 |
| 4,10 ± 0,05 | 5 |
| 3,90 ± 0,05 | 7 |
| 3,84 ± 0,05 | 8 |
| 3,71 ± 0,05 | 5 |
| 3,44 ± 0,05 | 30 |
| 3,34 ± 0,05 | 14 |
| 3,26 ± 0,05 | 9 |
| 3,08 ± 0,05 | 4 |
| 2,99 ± 0,05 | 3 |
| 2,89 ± 0,05 | 2 |
| 2,75 ± 0,05 | 1 |
| 2,37 ± 0,05 | 2 |
| 1,97 ± 0,05 | 2 |
| 1,86 ± 0,05 | 2 |

3. Zeolith nach Anspruch 1, worin die Kristallstsrukturen beschrieben werden können als: orthorhombisch mit Kristgallgitterkonstanten im Bereich von a = 18,35 ± 0,05 Å, b = 13,77 ± 0,03 Å, c = 7,37 ± 0,03 Å (Raumgruppe Pnma); orthorhombisch mit Gitterkonstanten im Bereich von a = 18,35 ± 0,05 Å, b = 13,77 ± 0,03 Å, c = 7,37 ± 0,03 Å (Raumgruppe Pnnm); orthorhombisch mit Gitterkonstanten im Bereich von a = 18,35 ± 0,05 Å, b = 13,77 ± 0,03 Å, c = 14,74 ± 0,03 Å (Raumgruppe Pbcm) oder monoklin mit Gitterkonstanten im Bereich von a = 18,35 ± 0,05 Å, b = 13,77 ± 0,03 Å, c = 7,37 ± 0,03 Å, β = 90 ± 0,3° (Raumgruppe P21/m).

4. Zeolith nach Anspruch 1, worin die lokale Koordination der O-Atome, welche die Si-Atome in der Skelettstruktur umgeben, vierfach koordiniert ist.

5. Zeolith nach Anspruch 1, worin die Skelettstruktur, die durch die Bindungen der Si-Atome und O-Atome gebikldet wird, über eine reguläre Geometrie verfügt.

6. Zeolith nach Anspruch 1, das über Poren mit einer mittleren Porenweite von 0,48 nm oder mehr verfügen, die mit Hilfe einer Methode der Gasadsorption gemessen wird.

7. Verfahren zum Herstellen eines Zeoliths, **gekennzeichnet durch** das Ausführen einer Wasser abspaltenden Polykondensation der mit kristallinen Schichten aufgebaute Verbindung oder der mit kristallinen Schichten aufgebaute Verbindung, die im Skelett substituierte Elemente enthält, wobei die mit kristallinen Schichten aufgebaute Verbindung eine chemische Zusammensetzung hat, die dargestellt wird **durch** [(Si₁₈₋ₓ0₃₈)M_{y}(TMA)_{z}(H₂O)_{w}] (worin TMA ein Kation Tetraalkylammonium ist, M ist ein Kation eines Alkalimetalls, das aus Na, K oder Li ausgewählt ist, x genügt der Gleichung 0 ≥ x ≥ 1,2, y genügt 0,5 ≥ y ≥ 1,5, z genügt 6 ≥ z ≥ 8 und w genügt 0,02 ≥ w ≥ 1,5), die als eine Grundstruktur daraus über ein einlagiges Skelett verfügt, welches eindimensionale Poren in Nanometergröße aufweist, die als ein Netzwerk von kovalenten Bindungen zwischen Si- und O-Atomen gebildet werden, wobei der Gitterabstand im Röntgenbeugungsdiagramm nach dem Pulververfahren mindestens Werte hat, wie sie in der nachfolgenden Tabelle 1 beschrieben werden (worin d der relative Gitterabstand ist, w ist eine schwache relative Intensität, m ist eine mittlere relative Intensität, s ist eine starke relative Intensität und vs ist eine sehr starke relative Intensität):
**Tabelle 1**
| d (Å) | relative Intensität |
|---|---|
| 10,47 ± 0,2 | vs |
| 8, 38 ± 0,15 | w |
| 7,34 ± 0,15 | m |
| 7,00 ± 0,1 | m |
| 6,51 ± 0,1 | m |
| 6,45 ± 0,1 | s |
| 5,86 ± 0,05 | m |
| 5,82 ± 0,04 | m |
| 5,66 ± 0,04 | w |
| 5,23 ± 0,04 | m |
| 5,07 ± 0,04 | w |
| 4,90 ± 0,04 | s |
| 4,75 ± 0,04 | m |
| 4,57 ± 0,04 | w |
| 4,40 ± 0,04 | m |
| 4,35 ± 0,04 | s |
| 4,26 ± 0,04 | s |
| 4,19 ± 0,04 | vs |
| 4,00 ± 0,04 | m |
| 3,94 ± 0,035 | s |
| 3,85 ± 0,035 | s |
| 3,83 ± 0,035 | vs |
| 3,78 ± 0,035 | w |
| 3,67 ± 0,035 | m |
| 3,63 ± 0,035 | s |
| 3,60 ± 0,035 | w |
| 3,55 ± 0,035 | m |
| 3,51 ± 0,035 | m |
| 3,50 ± 0,035 | vs |
| 3,48 ± 0,035 | vs |
| 3,38 ± 0,035 | m |
| 3,34 ± 0,035 | w |
| 3,32 ± 0,035 | s, |
um Zeolith synthetisch mit einer chemischen Zusammensetzung herzustellen, die dargestellt wird **durch** [(Sb₆₋ₓT_{y}O₇₂)M_{z}] (worin M ein Kation eines Alkalimetalls ist, das ausgewählt ist aus Li, Na, K oder Rb; T stellt Al, Ga oder Fe als Skelett substituierende Elemente dar; x genügt der Gleichung 0 ≥ x ≥ 3,0; y der Gleichung 0 ≥ y ≥ 1,0 und z der Gleichung 0 ≥ z ≥ 3,0).

8. Verfahren zum Herstellen eines Zeoliths nach Anspruch 7, bei welchem die Herstellung in einem Vakuum im Bereich von 1 x 10⁻³ bis 1 x 10⁻⁸ mmHg als eine Bedingung für eine Wasser abspaltende Polykondensation erfolgt.

9. Verfahren zum Herstellen eines Zeoliths nach Anspruch 7, bei welchem die Temperatur des Erhitzens der Wasser abspaltende Polykondensation 400° bis 800°C beträgt.

10. Verfahren zum Herstellen eines Zeoliths nach Anspruch 7, bei welchem das Zeolith bei Atmosphärendruck als Bedingung für eine Wasser abspaltende Polykondensation hergestellt wird.

11. Verfahren zum Herstellen eines Zeoliths nach Anspruch 7, bei welchem die Temperatur des Erhitzens der Wasser abspaltende Polykondensation 300° bis 800°C beträgt.

12. Verfahren zum Herstellen eines Zeoliths nach Anspruch 7, bei welchem die Geschwindigkeit des Temperaturanstiegs 0,5° bis 50°C pro Minute beträgt.

13. Verfahren zum Herstellen eines Zeoliths nach Anspruch 7, bei welchem die Wasser abspaltende Polykondensation ausgeführt wird mit einem Strom von Verbrennungsträgergas als Gas, das Sauerstoffmoleküle in einem molekularen Zustand aufweist.

14. Zeolith-Membran, **dadurch gekennzeichnet, dass** sie ein Zeolith (CDS-1) aufweist, das als eine Membran auf einem Träger gebildet wurde, wobei das Zeolith eine chemische Zusammensetzung hat, die dargestellt wird durch die Formel [(Si₃₆₋ₓO₇₂)M_{y}] (worin M ein Kation eines Alkalimetalls ist, das ausgewählt ist aus Na, K oder Li; x genügt der Gleichung 0 ≥ x ≥ 3,0 und y der Gleichung 0 ≥ y ≥ 3,0), sowie eine Struktur von Mikroporen hat, die aus kovalenten Bindungen zwischen Si- und O-Atomen aufgebaut wird und zwar mit einer Silicatstruktur von repetierenden Einheiten einer tetraedrischen Koordination von Si-O sowie geometrischen Kristallstrukturen (atomare Anordnung), die 5-gliedrige und 8-gliedrige Ringe aufweisen.

15. Zeolith-Membran nach Anspruch 14, worin die Kristallstrukturen sind:
(1) orthorhombisch mit Kristallgitterkonstanten im Bereich von a = 18,35 ± 0,05 Å, b = 13,77 ± 0,03 Å (Raumgruppe Pnma);
(2) orthorhombisch mit Gitterkonstanten im Bereich von a = 18,35 ± 0,05 Å, b = 13,77 ± 0,03 Å, c = 7,37 ± 0,03 Å (Raumgruppe Pnnm);
(3) orthorhombisch mit Gitterkonstanten im Bereich von a = 18,35 ± 0,05 Å, b = 13,77 ± 0,03 Å, c = 14,74 ± 0,03 Å (Raumgruppe Pbcm) oder
(4) monoklin mit Gitterkonstanten im Bereich von a = 18,35 ± 0,05 Å, b = 13,77 ± 0,03 Å, c = 7,37 ± 0,03 Å, β = 90 ± 0,3° (Raumgruppe P21/m).

16. Zeolith-Membran nach Anspruch 14, in welcher der Gitterabstand d (Å) im Röntgenbeugungsdiagramm nach dem Pulververfahren mindestens Werte hat, wie sie in den Tabellen 4 oder 5 beschrieben werden:
**Tabelle 4**
| d (Å) | relative Intensität |
|---|---|
| 9,17 ± 0,05 | 100 |
| 6,86 ± 0,05 | 35 |
| 6,11 ± 0,05 | 5 |
| 5,50 ± 0,05 | 4 |
| 4,84 ± 0,05 | 1 |
| 4,70 ± 0,05 | 1 |
| 4,58 ± 0,05 | 3 |
| 4,44 ± 0,05 | 7 |
| 4,35 ± 0,05 | 7 |
| 4,09 ± 0,05 | 6 |
| 3,88 ± 0,05 | 8 |
| 3,81 ± 0,05 | 9 |
| 3,68 ± 0,05 | 3 |
| 3,43 ± 0,05 | 16 |
| 3,41 ± 0,05 | 18 |
| 3,31 ± 0,05 | 8 |
| 3,24 ± 0,05 | 9 |
| 3,07 ± 0,05 | 1 |
**Tabelle 5**
| d (Å) | relative Intensität (Peak) |
|---|---|
| 9,25 ± 0,05 | 100 |
| 8,85 ± 0,05 | 7 |
| 7,67 ± 0,05 | 4 |
| 6,85 ± 0,05 | 65 |
| 6,14 ± 0,05 | 7 |
| 4,74 ± 0,05 | 6 |
| 4,65 ± 0,05 | 7 |
| 4,49 ± 0,05 | 13 |
| 4,40 ± 0,05 | 5 |
| 4,10 ± 0,05 | 5 |
| 3,90 ± 0,05 | 7 |
| 3,84 ± 0,05 | 8 |
| 3,71 ± 0,05 | 5 |
| 3,44 ± 0,05 | 30 |
| 3,34 ± 0,05 | 14 |
| 3,26 ± 0,05 | 9 |
| 3,08 ± 0,05 | 4 |
| 2,99 ± 0,05 | 3 |
| 2,89 ± 0,05 | 2 |
| 2,75 ± 0,05 | 1 |
| 2,37 ± 0,05 | 2 |
| 1,97 ± 0,05 | 2 |
| 1,86 ± 0,05 | 2 |

17. Zeolith-Membran nach Anspruch 14, in welcher der Träger eine poröse Basis aus einem anorganischen porösen Körper, Metall oder Metalloxid ist.

18. Verfahren zum Herstellen einer Zeolith-Membran, **gekennzeichnet durch** Verwenden eines kristallinen Schichtsilicats (nachfolgend abgekürzt als PLS) für Impfkristalle, dessen chemische Zusammensetzung dargestellt wird **durch** die Formel [(Si₁₈₋ₓO₃₈)M_{y}(TMA)_{z}(H₂O)_{w}] (worin TMA ein Kation Tetraalkylammonium ist, M ist ein Kation eines Alkalimetalls, das aus Na, K oder Li ausgewählt ist, x genügt der Gleichung 0 ≥ x ≥ 1,2, y genügt 0,5 ≥ y ≥ 1,5, z genügt 6 ≥ z ≥ 8 und w genügt 0,02 ≥ w ≥ 1,5), die als eine Grundstruktur daraus über ein einlagiges Silicat-Skelett verfügt, welches eindimensionale Poren in Nanometergröße aufweist, die als ein Netzwerk von kovalenten Bindungen zwischen Si- und O-Atomen gebildet werden; sowie Kondensieren der Si-OH-Gruppen in dem PLS zu CDS-1, das eine geometrische Kristallstruktur hat (atomare Anordnung), die 5-gliedrige und 8-gliedrige Ringe aufweist, sodass **dadurch** eine Zeolith-Membran auf einem Träger gebildet wird.

19. Verfahren zum Herstellen einer Zeolith-Membran nach Anspruch 18, bei welchem unter Verwendung von PLS als Impfkristalle eine PLS-Membran erzeugt wird.

20. Verfahren zum Herstellen einer Zeolith-Membran nach Anspruch 18, bei welchem der Träger eine poröse Basis aus einem anorganischen porösen Körper, Metall oder Metalloxid ist.

21. Verfahren zum Herstellen einer Zeolith-Membran nach Anspruch 19, bei welchem die PLS-Membran bis zu 300° bis 800°C erhitzt wird, um die Si-OH-Gruppen in dem PLS zu kondensieren und zu CDS-1 umzuwandeln.

22. Verfahren zum Herstellen einer Zeolith-Membran nach Anspruch 21, bei welchem die PLS-Membran unter vermindertem Druck erhitzt wird.

23. Verfahren zum Herstellen einer Zeolith-Membran nach Anspruch 19, bei welchem die PLS-Membran durch hydrothermale Synthese bei einer Temperatur von 140° bis 170°C erzeugt wird.

24. Verfahren zum Herstellen der CDS-1 Zeolith-Membran nach Anspruch 18, wobei die aus PLS synthetisch dargestellten Kristalle von CDS-1 zuerst auf einen Träger aufgebracht werden und anschließend eine Membran durch sekundäres Wachstum der Kristalle erzeugt wird.

25. Verfahren zum Herstellen von s-Caprolactam aus Cyclohexanonoxim-ε-Caprolactam, **dadurch gekennzeichnet, dass** ein Zeolith (CDS-1), das eine eine chemische Zusammensetzung hat, die dargestellt wird durch [(Si₃₆₋ₓT_{y}O₇₂)M_{z}] (worin M ein Kation eines Alkalimetalls ist, das ausgewählt ist aus Li, Na, K oder Rb; T stellt Al, Ga, Fe und Ce als Skelett substituierende Elemente dar; x genügt der Gleichung 0 ≥ x ≥ 3,0, y der Gleichung 0 ≥ y ≥ 1,0 und z der Gleichung 0 ≥ z ≥ 3,0), wobei die Zusammensetzung eine Struktur von Mikroporen aufweist, die aus kovalenten Bindungen zwischen den Atomden Si und O aufgebaut ist und eine geometrische Kristallstruktur hat (atomare Anordnung), die 5-gliedrige und 8-gliedrige Ringe aufweist und als Katalysator verwendet wird.

26. Verfahren zum Herstellen von ε-Caprolactam nach Anspruch 25, worin CDS-1 verwendet wird, das durch Wasser abspaltende Polykondensation bei Atmosphärendruck erhalten wurde.

27. Verfahren zum Herstellen von ε-Caprolactam nach Anspruch 25, worin CDS-1 verwendet wird, das durch Wasser abspaltende Polykondensation bei einer Temperatur des Erhitzens von 300° bis 800°C erhalten wurde.

28. Verfahren zum Herstellen von ε-Caprolactam nach Anspruch 25, worin CDS-1 verwendet wird, das durch Wasser abspaltende Polykondensation bei einer Geschwindigkeit des Temperaturanstiegs von 0,1° bis 10°C pro Minute erhalten wurde.

29. Verfahren zum Herstellen von ε-Caprolactam nach Anspruch 25, worin CDS-1 verwendet wird, das in der Synthese des CDS-1 durch Behandeln der mit kristallinen Schichten aufgebauten Silicatverbindung, die der Prekursor ist, mit einem Übergangsmetalloxid der Gruppe VI erhalten wurde.

30. Verfahren zum Herstellen von ε-Caprolactam nach Anspruch 25, worin der Gitterabstand d (Å) im Röntgenbeugungsdiagramm nach dem Pulververfahren des CDS-1 mindestens die in der nachfolgenden Tabelle 6 gegebenen Beugungspeaks zeigt:
**Tabelle 6**
| d (Å) | relative Intensität (Peak) |
|---|---|
| 9,17 ± 0,05 | 100 |
| 6,86 ± 0,05 | 35 |
| 6,11 ± 0,05 | 5 |
| 5,50 ± 0,05 | 4 |
| 4,58 ± 0,05 | 3 |
| 4,44 ± 0,05 | 7 |
| 4,35 ± 0,05 | 7 |
| 4,09 ± 0,05 | 6 |
| 3,88 ± 0,05 | 8 |
| 3,81 ± 0,05 | 9 |
| 3,68 ± 0,05 | 3 |
| 3,43 ± 0,05 | 16 |
| 3,41 ± 0,05 | 18 |
| 3,31 ± 0,05 | 8 |
| 3,24 ± 0,05 | 9 |

31. Verfahren zum Herstellen von ε-Caprolactam nach Anspruch 25, worin das CDS-1 auf der Grundlage der physikalischen Adsorption Mikroporen mit einer mittleren Porenweite von 0,483 nm oder mehr hat und ein Volumen von 0,6 cm³/g oder mehr.

32. Verfahren zum Herstellen von ε-Caprolactam nach Anspruch 25, worin das CDS-1, das in der Umlagerungsreaktion nach Beckmann zur Anwendung gelangt, einen Kationenaustausch oder Wasserstoffionenaustausch erfahren hat.

33. Verfahren zum Herstellen von ε-Caprolactam nach Anspruch 25, worin die Reaktionstemperatur in der Herstellung von -Caprolactam aus Cyclohexanonoxim 150° bis 500°C beträgt.

34. Verfahren zum Herstellen von ε-Caprolactam nach Anspruch 25, worin das der ständliche Massedurchsatz, WHSV, des Cyclohexanonoxims zwischen 0,001 h¹ und 20,0 h¹ beträgt.

## Revendications

1. Zéolite **caractérisée en ce qu'**elle a la composition chimique (composition structurelle des cristaux) représentée par [(Si₃₆₋ₓT_{y}O₇₂)M_{z}] (où M est un cation d'un métal alcalin choisi parmi Li, Na, K ou Rb, T représente Al, Ga ou FE comme éléments substituants de squelette, x satisfait 0 ≤ x ≤ 3,0, y satisfait 0 ≤ y ≤ 1,0 et z satisfait 0 ≤ z ≤ 3,0), et ayant une structure de micropores constituée de liaisons covalentes entre les atomes Si et O.

2. Zéolite selon la revendication 1, dans laquelle la constante réticulaire d(Å) dans le diagramme de diffraction de rayons x sur poudre est comme décrit au tableau 2 ou 3 ci-dessous.
**Tableau 2**
| d(Å) | Force relative |
|---|---|
| 9,17 ± 0,05 | 100 |
| 6,86 ± 0,05 | 35 |
| 6,11 ± 0,05 | 5 |
| 5,50 ± 0,05 | 4 |
| 4,84 ± 0,05 | 1 |
| 4,70 ± 0,05 | 1 |
| 4,58 ± 0,05 | 3 |
| 4,44 ± 0,05 | 7 |
| 4,35 ± 0,05 | 7 |
| 4,09 ± 0,05 | 6 |
| 3,88 ± 0,05 | 8 |
| 3,81 ± 0,05 | 9 |
| 3,68 ± 0,05 | 3 |
| 3,43 ± 0,05 | 25 |
| 3,41 ± 0,05 | 29 |
| 3,31 ± 0,05 | 8 |
| 3,24 ± 0,05 | 9 |
| 3,07 ± 0,05 | 1 |
**Tableau 3**
| d(Å) | Force relative |
|---|---|
| 9,25 ± 0,05 | 100 |
| 8,85 ± 0,05 | 7 |
| 7,67 ± 0,05 | 4 |
| 6,14 ± 0,05 | 7 |
| 6,85 ± 0,05 | 65 |
| 4,74 ± 0,05 | 6 |
| 4,65 ± 0,05 | 7 |
| 4,49 ± 0,05 | 13 |
| 4,40 ± 0,05 | 5 |
| 4,10 ± 0,05 | 5 |
| 3,90 ± 0,05 | 7 |
| 3,84 ± 0,05 | 8 |
| 3,71 ± 0,05 | 5 |
| 3,44 ± 0,05 | 30 |
| 3,34 ± 0,05 | 14 |
| 3,26 ± 0,05 | 9 |
| 3,08 ± 0,05 | 4 |
| 2,99 ± 0,05 | 3 |
| 2,89 ± 0,05 | 2 |
| 2,75 ± 0,05 | 1 |
| 2,37 ± 0,05 | 2 |
| 1,97 ± 0,05 | 2 |
| 1,86 ± 0,05 | 2 |

3. Zéolite selon la revendication 1, dans laquelle les structures cristallines peuvent être décrites comme orthorhombiques avec des constantes de réseau cristallin dans la plage de a = 18,35 ± 0,05 Å, b = 13,77 ± 0,03, c = 7,37 ± 0,03 Å (groupe spatial Pnma),
orthorhombiques avec des constantes de réseau cristallin dans la plage de a = 18,35 ± 0,05 Å, b = 13,77 ± 0,03, c = 7,37 ± 0,03 Å (groupe spatial Pnnm), orthorhombiques avec des constantes de réseau cristallin dans la plage de a = 18,35 ± 0,05 Å, b = 13,77 ± 0,03, c = 14,74 ± 0,03 Å (groupe spatial Pbcm) ou monocliniques avec des constantes de réseau cristallin dans la plage de a = 18,35 ± 0,05 Å, b = 13,77 ± 0,03, c = 7,37 ± 0,03 Å, β = 90 ± 0,3° (groupe spatial P21/m).

4. Zéolite selon la revendication 1, dans laquelle la coordination locale des atomes 0 entourant les atomes Si dans la structure squelettique est tetracoordonnée.

5. Zéolite selon la revendication 1, dans laquelle la structure squelettique formée par la liaison des atomes Si et 0 a une géométrie régulière.

6. Zéolite selon la revendication 1, ayant des pores avec une taille moyenne de 0,48 nm ou plus mesurée par un procédé d'adsorption de gaz.

7. Procédé de fabrication d'une zéolite comprenant les étapes consistant à effectuer une polycondensation avec déshydratation du composé cristallin stratifié ou du composé cristallin stratifié contenant des éléments substitués squelettiques, ledit composé cristallin stratifié ayant une composition chimique représentée par [(Si₁₈₋ₓO₃₈)M_{y} (TMA)_{z} (H₂O)_{w}] (où TMA est un cation tétraalkylammonium, M est un cation d'un métal alcalin choisi parmi Na, K ou Li, x satisfait 0 ≤ x ≤ 1,2, y satisfait 0,5 ≤ y ≤ 1,5, z satisfait 6 ≤ z ≤ 8 et w satisfait 0,02 ≤ w ≤ 1,5), ayant comme structure de base de celle-ci un squelette à couche unique comprenant des nanomètres de micropores unidimensionnels en taille formés par un réseau de liaisons covalentes entre atomes Si et O, la constante réticulaire d dans le diagramme de diffraction aux rayons x sur poudre étant au moins comme décrit au tableau 1 ci-dessous (où d est la constante réticulaire, w = force relative faible, m = force relative modérée, s = force relative forte et vs = force relative très forte) :
**Tableau 1**
| d(Å) | Force relative |
|---|---|
| 10,47 ± 0,2 | vs |
| 8,38 ± 0,15 | w |
| 7,34 ± 0,15 | m |
| 7,00 ± 0,1 | m |
| 6,51 ± 0,1 | m |
| 6,45 ± 0,1 | s |
| 5,86 ± 0,05 | m |
| 5,82 ± 0,04 | m |
| 5,66 ± 0,04 | w |
| 5,23 ± 0,04 | m |
| 5,07 ± 0,04 | w |
| 4,90 ± 0,04 | s |
| 4,75 ± 0,04 | m |
| 4,57 ± 0,04 | w |
| 4,40 ± 0,04 | m |
| 4,35 ± 0,04 | s |
| 4,26 ± 0,04 | s |
| 4,19 ± 0,04 | vs |
| 4,00 ± 0,04 | m |
| 3,94 ± 0,035 | s |
| 3,85 ± 0,035 | s |
| 3,83 ± 0,035 | vs |
| 3,78 ± 0,035 | w |
| 3,67 ± 0,035 | m |
| 3,63 ± 0,035 | s |
| 3,60 ± 0,035 | w |
| 3,55 ± 0,035 | m |
| 3,51 ± 0,035 | m |
| 3,50 ± 0,035 | vs |
| 3,48 ± 0,035 | vs |
| 3,38 ± 0,035 | m |
| 3,34 ± 0,035 | w |
| 3,32 ± 0,035 | s, |
pour synthétiser une zéolite avec la composition chimique représentée par [(Si₃₆₋ₓT_{y}O₇₂)M_{z}] (où M est un cation d'un métal alcalin choisi parmi Li, Na, K ou Rb, T représente Al, Ga ou Fe comme éléments substituants de squelette, x satisfait 0 ≤ x ≤ 3,0, y satisfait 0 ≤ y ≤ 1,0 et z satisfait 0 ≤ z ≤ 3,0).

8. Procédé de fabrication d'une zéolite selon la revendication 7, dans lequel la fabrication s'effectue dans un vide dans la plage de 1 x 10⁻³ à 1 x 10⁻⁸ torr comme condition pour la polycondensation avec déshydratation.

9. Procédé de fabrication d'une zéolite selon la revendication 7, dans lequel la température de chauffage pour la polycondensation avec déshydratation est comprise entre 400 et 800 °C.

10. Procédé de fabrication d'une zéolite selon la revendication 7, dans lequel la zéolite est fabriquée à la pression atmosphérique comme condition pour la polycondensation avec déshydratation.

11. Procédé de fabrication d'une zéolite selon la revendication 7, dans lequel la température de chauffage pour la polycondensation avec déshydratation est comprise entre 300 et 800 °C.

12. Procédé de fabrication d'une zéolite selon la revendication 7, dans lequel le taux de hausse de la température est compris entre 0,5 et 50 °C par minute.

13. Procédé de fabrication d'une zéolite selon la revendication 7, dans lequel la polycondensation avec déshydratation est effectuée avec un flux de gaz soutenant la combustion, un gaz comprenant des molécules d'oxygène dans un état moléculaire.

14. Membrane de zéolite **caractérisée en ce qu'**elle comprend une zéolite (CDS-1) formée comme une membrane sur un support, ladite zéolite ayant la composition chimique représenté par [(Si₃₆₋ₓO₇₂)M_{y}] (où M est un cation d'un métal alcalin choisi parmi Na, K ou Li, x satisfait ≤ x ≤ 3,0, y satisfait 0 ≤ y ≤3,0) et une structure de micropores constituée de liaisons covalentes entre atomes Si et 0, avec une structure de silicate d'unités répétées de coordination tétrahédrale Si-O et des structures cristallines géométriques (disposition atomique) comprenant des anneaux de 5 atomes et 8 atomes de silicium.

15. Membrane de zéolite selon la revendication 14, dans laquelle les structures cristallines sont (1) orthorhombiques avec des constantes de réseau cristallin dans la plage de a = 18,35 ± 0,05 Å, b = 13,77 ± 0,03, c = 7,37 ± 0,03 Å (groupe spatial Pnma), (2) orthorhombiques avec des constantes de réseau cristallin dans la plage de a = 18,35 ± 0,05 Å, b = 13,77 ± 0,03, c = 7,37 ± 0,03 Å (groupe spatial Pnnm), (3) orthorhombiques avec des constantes de réseau cristallin dans la plage de a = 18,35 ± 0,05 Å, b = 13,77 t 0,03, c = 14,74 ± 0,03 Å (groupe spatial Pbcm) ou (4) monocliniques avec des constantes de réseau cristallin dans la plage de a = 18,35 ± 0,05 Å, b = 13,77 ± 0,03, c = 7,37 ± 0,03 Å, p = 90 ± 0,3° (groupe spatial P21/m).

16. Membrane de zéolite selon la revendication 14, dans laquelle la constante réticulaire d(Å) dans le diagramme de diffraction aux rayons x sur poudre est comme décrit aux tableaux 4 et 5.
**Tableau 4**
| d(Å) | Force relative |
|---|---|
| 9,17 ± 0,05 | 100 |
| 6,86 ± 0,05 | 35 |
| 6,11 ± 0,05 | 5 |
| 5,50 ± 0,05 | 4 |
| 4,84 ± 0,05 | 1 |
| 4,70 ± 0,05 | 1 |
| 4,58 ± 0,05 | 3 |
| 4,44 ± 0,05 | 7 |
| 4,35 ± 0,05 | 7 |
| 4,09 ± 0,05 | 6 |
| 3,88 ± 0,05 | 8 |
| 3,81 ± 0,05 | 9 |
| 3,68 ± 0,05 | 3 |
| 3,43 ± 0,05 | 16 |
| 3,41 ± 0,05 | 18 |
| 3,31 ± 0,05 | 8 |
| 3,24 ± 0,05 | 9 |
| 3,07 ± 0,05 | 1 |
**Tableau 5**
| d(Å) | Force relative |
|---|---|
| 9,25 ± 0,05 | 100 |
| 8,85 ± 0,05 | 7 |
| 7,67 ± 0,05 | 4 |
| 6,85 ± 0,05 | 65 |
| 6,14 ± 0,05 | 7 |
| 4,74 ± 0,05 | 6 |
| 4,65 ± 0,05 | 7 |
| 4,49 ± 0,05 | 13 |
| 4,40 ± 0,05 | 5 |
| 4,10 ± 0,05 | 5 |
| 3,90 ± 0,05 | 7 |
| 3,84 ± 0,05 | 8 |
| 3,71 ± 0,05 | 5 |
| 3,44 ± 0,05 | 30 |
| 3,34 ± 0,05 | 14 |
| 3,26 ± 0,05 | 9 |
| 3,08 ± 0,05 | 4 |
| 2,99 ± 0,05 | 3 |
| 2,89 ± 0,05 | 2 |
| 2,75 ± 0,05 | 1 |
| 2,37 ± 0,05 | 2 |
| 1,97 ± 0,05 | 2 |
| 1,86 ± 0,05 | 2 |

17. Membrane de zéolite selon la revendication 14, dans laquelle le support est une base poreuse d'un corps poreux inorganiques, métal ou oxyde métallique.

18. Procédé de fabrication de membrane de zéolite comprenant les étapes consistant à utiliser comme germes cristallins un silicate cristallin stratifié (ci-après abrégé en PLS), dont la composition chimique est représentée par [(Si_{18_x}O₃₈) M_{y}(TMA)_{z} (H₂O)_{w}] (où TMA est un cation tétraalkylammonium, M est un cation d'un métal alcalin, x satisfait 0 ≤ x ≤ 1,2, y satisfait 0,5 ≤ y ≤ 1,5, z satisfait 6 ≤ z ≤ 8 et w satisfait 0,02 ≤ w ≤ 1,5 ), et ayant comme structure de base un squelette de silicate à couche unique comprenant des nanomètres de micropores unidimensionnels de taille formés par un réseau de liaisons covalentes entre atomes Si et O, condenser les groupes Si-OH dans le PLS pour convertir le PLS en CDS-1 1 ayant une structure cristalline géométrique (disposition atomique) comprenant des anneaux à 5 atomes et à 8 atomes de silicium, et former ainsi une membrane de zéolite sur un support.

19. Procédé de fabrication de membrane de zéolite selon la revendication 18, dans lequel une membrane PLS est formée en utilisant des germes cristallins PLS.

20. Procédé de fabrication de membrane de zéolite selon la revendication 18, dans lequel le support est une base poreuse d'un corps poreux inorganique, métal ou oxyde métallique.

21. Procédé de fabrication de membrane de zéolite selon la revendication 19, dans lequel la membrane PLS est chauffée entre 300 et 800 °C pour condenser les groupes Si-OH dans le PLS et les convertir en CDS-1.

22. Procédé de fabrication de membrane de zéolite selon la revendication 21, dans lequel la membrane PLS est chauffée sous pression réduite.

23. Procédé de fabrication de membrane de zéolite selon la revendication 19, dans lequel la membrane PLS est formée par synthèse hydrothermique à une température comprise entre 140 et 170 °C.

24. Procédé de fabrication de membrane de zéolite CDS-1 selon la revendication 18, dans lequel les cristaux CDS-1 synthétisés à partir de PLS sont tout d'abord appliqués sur un support, et une membrane est ensuite formée par croissance secondaire des cristaux.

25. Procédé de fabrication de ε-caprolactame à partir de ε-caprolactame d'oxime de cyclohexanone, **caractérisé en ce qu'**une zéolite (CDS-1) ayant la composition chimique représentée par [(S₁₃₆₋ₓT_{y}O₇₂)M_{z}] (où M est un cation d'un métal alcalin choisi parmi Li, Na, K ou Rb, T représente Al, Ga, Fe et Ce comme éléments substituants de squelette, x satisfait 0 ≤ x ≤ 3,0, y satisfait 0 < y ≤ 1,0 et z satisfait 0 ≤ z ≤ 3,0), et ayant une structure de micropores constituée de liaisons covalentes entre les atomes Si et O et une structure cristalline géométrique (disposition atomique) comprenant des anneaux à 5 atomes et à 8 atomes de silicium est utilisée comme catalyseur.

26. Procédé de fabrication de ε-caprolactame selon la revendication 25, dans lequel le CDS-1 obtenu par polycondensation avec déshydratation à la pression atmosphérique est utilisé.

27. Procédé de fabrication de ε-caprolactame selon la revendication 25, dans lequel le CDS-1 obtenu par polycondensation avec déshydratation à une température de chauffage de 300 à 800 °C est utilisé.

28. Procédé de fabrication de ε-caprolactame selon la revendication 25, dans lequel le CDS-1
obtenu par polycondensation avec déshydratation avec un taux de hausse de température de 0,1 à 10 °C/minute est utilisé.

29. Procédé de fabrication de ε-caprolactame selon la revendication 25, dans lequel le CDS-1 obtenu par traitement du composé de silicate cristallin stratifié qui est le précurseur avec un oxyde métallique transitoire de groupe 6 dans le processus de synthèse CDS-1 est utilisé.

30. Procédé de fabrication de ε-caprolactame selon la revendication 25, dans lequel la constante réticulaire d(Å) dans le diagramme de diffraction aux rayons x sur poudre du CDS-1 présente au moins les pics de diffraction indiqués au tableau 6 ci-dessous.
**Tableau 6**
| d(Å) | Force relative |
|---|---|
| 9,17 ± 0,05 | 100 |
| 6,86 ± 0,05 | 35 |
| 6,11 ± 0,05 | 5 |
| 5,50 ± 0,05 | 4 |
| 4,58 ± 0,05 | 3 |
| 4,44 ± 0,05 | 7 |
| 4,35 ± 0,05 | 7 |
| 4,09±0,05 | 6 |
| 3,88±0,05 | 8 |
| 3,81±0,05 | 9 |
| 3,68±0,05 | 3 |
| 3,43±0,05 | 16 |
| 3,41 ± 0,05 | 18 |
| 3,31 ± 0,05 | 8 |
| 3,24 ± 0,05 | 9 |

31. Procédé de fabrication de ε-caprolactome selon la revendication 25, dans lequel le CDS-1 a des micropores avec une taille de pore moyenne de 0,483 nm ou plus en fonction de l'adsorption physique et un volume de 0,6 cc/g ou plus.

32. Procédé de fabrication de ε-caprolactame selon la revendication 25, dans lequel le CDS-1 utilisé dans la réaction de la transposition de Beckmann est échangé par des cations ou échangé par des ions d'hydrogène.

33. Procédé de fabrication de ε-caprolactame selon la revendication 25, dans lequel la température de réaction dans le procédé de fabrication de ε-caprolactame à partir d'oxime de cyclohexanone est comprise entre 150 et 500 °C.

34. Procédé de fabrication de ε-caprolactame selon la revendication 25, dans lequel le WHSV de l'oxime de cyclohexanone est compris entre 0,001 h⁻¹ et 20,0 h⁻¹.
